# FASCICULE DE BREVET EUROPEEN

(11) **EP 1 017 681 B1**
(45) Date de publication et mention de la délivrance du brevet: **09.04.2003**
(21) Numéro de dépôt: 98945363.4
(22) Date de dépôt: 23.09.1998
(51) Int. Cl.: C07D 233/52, A61K 31/415, C07C 49/747, C07C 333/04, C07C 323/22

(54) **DERIVES D'HYDRAZONO-BENZAZULENE, COMPOSITIONS PHARMACEUTIQUES ET INTERMEDIAIRES**
HYDRAZONO-BENZAZULEN-DERIVATE, PHARMAZEUTISCHE ZUSAMMENSETZUNGEN UND ZWISCHENPRODUKTE
HYDRAZONO-BENZAZULENE DERIVATIVES, PHARMACEUTICAL COMPOSITIONS AND INTERMEDIATES

(30) Priorité: 24.09.1997 FR 9711859
(43) Date de publication de la demande: 12.07.2000
(73) Titulaire: Aventis Pharma S.A., 92160 Antony (FR); GENENTECH, INC., South San Francisco, CA 94080-4990 (US)
(72) Inventeur: CARNIATO, Denis, F-91460 Marcoussis (FR); GADEK, Thomas, R., Oakland, CA 94611 (US); GOUBET, François, F-75015 Paris (FR); GOURVEST, Jean-François, F-77140 Claye-Souilly (FR); KNOLLE, Jochen, South San Francisco, CA 94080 (US); MCDOWELL, Robert, San Francisco, CA 94114 (US); SCHEUNEMANN, Karl-Heinz, D-65835 Liederbach (DE)
(86) Numéro de dépôt international: FR9802039
(87) Numéro de publication internationale: WO99015507

(56) Documents cités:
- EP-A- 0 729 933
- EP-A- 0 820 988
- WO-A-96/06087
- WO-A-97/34865

## Description

La présente invention concerne de nouveaux composés tricycliques, leur procédé de préparation et les intermédiaires de ce procédé, leur application à titre de médicaments et les compositions pharmaceutiques les renfermant.

La présente invention a pour objet les composés de formule générale (I) : 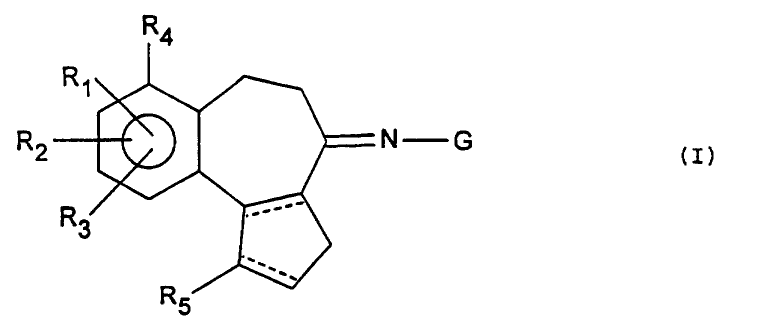 dans laquelle R₁ représente un groupement -S(O)ₓ-[A]-[B]-COR₆, x étant égal à 0, 1 ou 2, -[A]- représentant un radical bivalent alkylène, alkénylène ou alkynylène, linéaire ou ramifié, comportant de 1 à 6 atomes de carbone,
[B] représentant un radical phényle, un radical CH(Z), ou une simple liaison,
(Z) représente un atome d'hydrogène, un groupement
(CH₂)₀₋₆-NRaRb, (CH₂)₀₋₆-NH-SO₂-Rc, (CH₂)₀₋₆-NH-CO₂-Rc,
(CH₂)₀₋₆-NH-CO-Rc, (CH₂)₀₋₆-NH-SO₂-NH-Rc, (CH₂)₀₋₆-NH-CO-NH-Rc,
(CH₂)₀₋₆-CO₂-Rc, (CH₂)₀₋₆-SO₂-Rc, (CH₂)₀₋₆-CO-Rc ou (CH₂)₀₋₆-Rc dans lesquels (CH₂)₀₋₆ est un radical bivalent alkylène comportant de 1 à 6 atomes de carbone, ou une simple liaison dans le cas de (CH₂)₀,
Ra, Rb et Rc représentent un atome d'hydrogène, un radical (CH₂)₀₋₃-Ar dans lequel Ar représente un groupement phényle ou un radical (CH₂)₀₋₃-Alk dans lequel Alk représente un radical alkyle, alkényle ou alkynyle, linéaire, ramifié ou cyclique, et comportant de 1 à 12 atomes de carbone, les radicaux Ar et Alk pouvant être non substitués ou substitués par
- halogène
- alkyle, alkényle, alkynyle renfermant de 1 à 12 atomes de carbone,
- oxo, cyano, nitro, formyl, carboxy et carboxyalkyle renfermant de 1 à 6 atomes de carbone, carboxamide,
- alkoxy renfermant de 1 à 12 atomes de carbone,
- alkylthio renfermant de 1 à 12 atomes de carbone,
- amino, alkylamino renfermant de 1 à 12 atomes de carbone, dialkylamino renfermant de 2 à 24 atomes de carbone,
- aminoalkyle renfermant de 1 à 12 atomes de carbone,
- dialkylaminoalkyle renfermant de 3 à 25 atomes de carbone,
- dialkylaminoalkyloxy renfermant de 3 à 25 atomes de carbone,
- hydroxyle éventuellement acylé renfermant de 1 à 12 atomes de carbone,
- acyle renfermant de 1 à 12 atomes de carbone ou benzoyle éventuellement substitués par un atome de chlore, d'iode ou de fluor,
- phényle, furyle, thiényle, pyridinyle ou benzyle éventuellement substitués par halogène, alkyle, alkoxy, alkylthio, aminoalkyle ou dialkylamino indiqués ci-dessus, ou encore, Ra et Rb représentent ensemble avec l'atome d'azote auquel ils sont liés un hétérocycle choisis parmi morpholine, pipéridine, pipérazine, pyrrolidine, pyrimidine, pyridine ou pyrazine,
- R₆ représente un groupement -OH, -OCH₃, -OCH₂CH₃, -O-(CH₂)₂-OH, -O-CH₂-CH(OH)-CH₂OH, -O-(CH₂)₂-NH₂, -O- (CH₂)₂-N-(CH₃)₂, -NH₂ ou -O-(CH₂)-phényle,
- R₂ et R₃ identiques ou différents représentent un atome d'hydrogène, un radical hydroxyle ou un radical O-Me,
- R₄ représente un atome d'hydrogène,
- R₅ représente un atome d'hydrogène,
- G représente,
**soit** un radical de formule G1 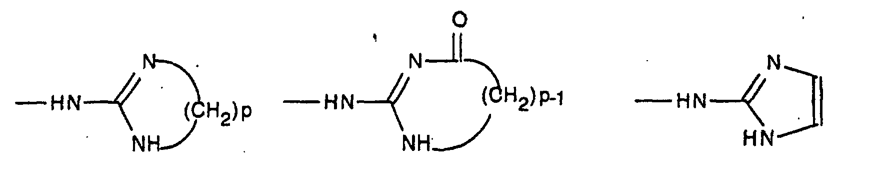 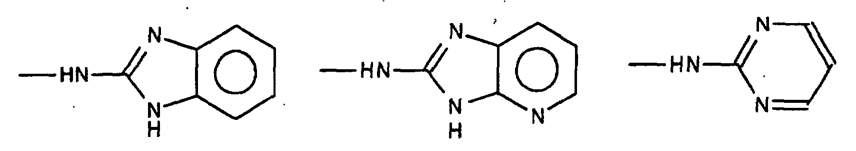 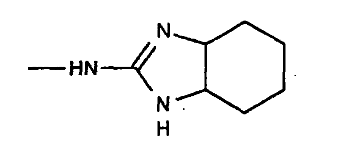 p étant un entier égal à 2, 3 ou 4,
- **soit** un radical NRaRb (radical G2), Ra et Rb étant tels que définis plus haut,
- **soit** un radical -NH-C(=X)-NHRc (radical G4), dans lequel X est un atome de soufre, d'oxygène ou NH et Rc est tel que défini précédemment,
- **soit** un radical -NH-SO₂Rc (radical G5), dans lequel Rc est tel que défini précédemment,
les traits en pointillés représentent une éventuelle seconde liaison, ainsi que les sels d'addition avec les acides ou les bases,
R₁, R₂ et R₃ peuvent être en position 8, 9 ou 10 du tricycle.

Par composé de formule (I) on désigne tous les isomères géométriques et les stéréoisomères possibles pris individuellement ou en mélange.

-[A]- représente notamment les radicaux alkylènes tels que -CH₂-, -CH₂CH₂-, -CH₂CH₂CH₂- ou -CH₂CH₂CH₂CH₂-, ou bien les radicaux alkénylène ou alkynylène tels que -CH=CH-CH₂- ou -C≡C-CH₂-.

Lorsque ces radicaux bivalents sont ramifiés, il peut s'agir de radicaux tels que -CH(CH₃)-, -C(Me)₂, -CH₂-C(Me)₂-, -CH(Et)-, -CH(C≡CH)- ou -C(C≡CH) (Et)-.

Lorsque [B] représente un radical bivalent -Ph-, le groupement COR₆ peut être en position ortho, méta ou para. Il se trouve de préférence en position para.

(CH₂)₀₋₆ sera de préférence une simple liaison ou un groupement (CH₂)ₙ, n étant un entier choisi parmi 1, 2 ou 3.

Lorsque Ra, Rb et Rc représentent un groupement (CH₂)₀₋₃-Ar, (CH₂)₀₋₃-Alk, (CH₂)₀₋₃ représente soit une simple liaison dans le cas de (CH₂)₀, soit les radicaux -CH₂-, -(CH₂)₂- ou -(CH₂)₃-.

Par le terme Alk on désigne les radicaux alkyles tels que méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle, les radicaux alkényles tels que vinyle, propényle, isopropényle, allyle, 2-méthylallyle, butényle ou isobutényle, les radicaux alkynyles tels que éthynyle, propynyle, propargyle, butynyle ou isobutynyle, et les radicaux cycloalkyles, tels que cyclopropyle, cyclobutyle, cyclopentyle, cyclohexyle ou adamantyle.

G2 peut être notamment un groupement NH₂, NH-Alk tel que NHMe, NHEt, N(Alk)₂ tel que NMe₂, NEt₂, NMeEt, NH-(CH₂)₀₋₁-Ar tel que NHPh, NHCH₂Ph.
- Lorsque G est un radical -NH-C(=X)-NHRc (radical G4), ou NHSO₂Rc (radical G5), dans lesquels X est un atome de soufre, d'oxygène ou NH, et Rc est tel que défini précédemment. Il s'agit notamment des groupements -NH-C(=NH)-NH₂, -NH-C(=O)-NH₂ ou -NH-C(=S)-NH₂, -NH-C(=NH)-NHCH₂-Ar tel que -NH-C(=NH)-NHCH₂Ph, -NH-C(=NH)-NH-Alk tel que -NH-C(=NH)-NHCH₃, ou -NH-SO₂Ph, les groupements Ar ou Alk étant substitués ou non substitués.

Les substituants éventuels des radicaux Alk ou Ar sont de préférence les radicaux suivants :
- halogène : fluor, chlore, brome, iode,
- alkyle, alkényle, alkynyle renfermant de 1 à 12 atomes de carbone tel que méthyle, éthyle, propyle, isopropyle, butyle, isobutyle, tert-butyle, vinyl ou allenyl,
- oxo, cyano, nitro, formyl, carboxy et carboxyalkyl renfermant de 1 à 6 atomes de carbone, carboxamide,
- alkoxy renfermant de 1 à 12 atomes de carbone tel que méthoxy, éthoxy, propyloxy, isopropyloxy, butyloxy,
- alkylthio renfermant de 1 à 12 atomes de carbone tel que méthylthio, éthylthio, propylthio, isopropylthio, butylthio,
- amino, alkylamino renfermant de 1 à 12 atomes de carbone tel que méthylamino ou éthylamino, dialkylamino renfermant de 2 à 24 atomes de carbone tel que diméthylamino, diéthylamino, méthyléthylamino,
- aminoalkyle renfermant de 1 à 12 atomes de carbone tel que aminométhyle ou aminoéthyle,
- dialkylaminoalkyle renfermant de 3 à 25 atomes de carbone tel que diméthylamino méthyle ou éthyle,
- dialkylaminoalkyloxy renfermant de 3 à 25 atomes de carbone tel que diméthylaminoéthyloxy,
- hydroxyle éventuellement acylé renfermant de 1 à 12 atomes de carbone, par exemple acétoxy,
- acyle renfermant de 1 à 12 atomes de carbone tel que formyle, acétyle, propionyle, butyryle, isobutyryle, valéryle, isovaléryle, succinyle, pivaloyle, benzoyle, éventuellement substitué par un atome de chlore, d'iode ou de fluor. On peut citer les radicaux chloroacétyle, dichloroacétyle, trichloroacétyle, bromoacétyle ou trifluoroacétyle,
- phényle, furyle, thiènyle, pyridinyle ou benzyle, ces radicaux étant eux-mêmes éventuellement substitués par des radicaux halogène, alkyle, alkoxy, alkylthio, amino alkyle ou dialkylamino indiqués ci-dessus.

Bien entendu, un ou plusieurs substituants, identiques ou différents, peuvent être présents.

Il est bien entendu que entendu que lorsque Rₐ, R_{b}, R_{c} représentent un groupement alkyle ou aryle tels que définis ci-dessus, ils peuvent être identiques ou différents indépendamment les uns des autres.

L'invention s'étend naturellement aux sels des composés de formule (I), comme par exemple les sels formés, lorsque les composés de formule (I) comportent une fonction amino ou amino guanidine, avec les acides chlorhydrique, bromhydrique, nitrique, sulfurique, phosphorique, acétique, trifluoroacétique, formique, propionique, benzoïque, maléique, fumarique, succinique, tartrique, citrique, oxalique, glyoxylique, aspartique, alcanesulfoniques tels que les acides méthane ou éthanesulfonique, arènesulfoniques, tels que les acides benzène ou paratoluène sulfonique et arylcarboxylique, ou lorsque les composés de formule (I) comportent une fonction acide, avec les sels des métaux alcalins ou alcalino terreux ou d'ammonium éventuellement substitué.

Dans un premier groupe préféré, l'invention a pour objet les composés de formule générale (I) telle que définie précédemment, répondant à la formule générale (I') : 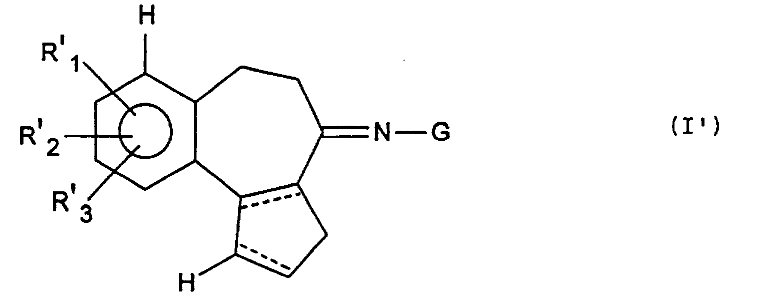 dans laquelle R'₁ représente un groupement
-S(O)ₓ-[A']-[B']-COR'_{6,} x étant égal à 0, 1 ou 2, -[A']- représentant un radical bivalent alkylène, alkénylène ou alkynylène renfermant de 2 à 6 atomes de carbone, [B'] représentant un radical CH(Z') ou une simple liaison,
(Z') représente un atome d'hydrogène, un groupement
(CH₂)₀₋₆-NRaRb, (CH₂)₀₋₆-NH-SO₂-Rc, (CH₂)₀₋₆-NH-CO₂-Rc, (CH₂)₀₋₆-NH-CO-Rc, (CH₂)₀₋₆-NH-SO₂-NH-Rc, (CH₂)₀₋₆-NH-CO-NH-Rc, (CH₂)₀₋₆-CO₂-Rc, (CH₂)₀₋₆-SO₂-Rc, (CH₂)₀₋₆-CO-Rc ou (CH₂)₀₋₆-Rc, Ra, Rb et Rc étant tels que définis précédemment, R'₆ étant R₆ tel que défini précédemment R'₂ et R'₃ représentent un atome d'hydrogène ou un radical méthoxy, et G est tel que défini précédemment, les traits en pointillés représentent une éventuelle seconde liaison, ainsi que les sels d'addition avec les acides ou les bases.

Dans un deuxième groupe préféré l'invention a pour objet les composés de formule générale (I) telle que définie précédemment dans laquelle R₆ représente un groupement -OH, -OCH₃, ainsi que les sels d'addition avec les acides ou les bases.

Dans un troisième groupe préféré, l'invention a pour objet les composés de formule générale (I) telle que définie précédemment dans laquelle R₁ représente un groupement S(O)ₓ-(CH₂)₂-CH(Z')-COOH, ainsi que les sels d'addition avec les acides ou les bases.

Dans un quatrième groupe préféré, l'invention a pour objet les composés de formule générale (I) telle que définie précédemment, dans laquelle (Z') est le groupement (CH₂)₀₋₆-NH-CO₂-Rc ou (CH₂)₀₋₆-NHRb, Rb et Rc étant tels que définis à précédemment, ainsi que les sels d'addition avec les acides ou les bases.

Dans un cinquième groupe préféré, l'invention a pour objet les composés de formule générale (I) telle que définie précédemment, dans laquelle (Z') est le groupement MHCO₂Rc ou NHRb, ainsi que les sels d'addition avec les acides ou les bases.

Dans un sixième groupe préféré, l'invention a pour objet les composés de formule générale (I) telle que définie précédemment, dans laquelle Rb et Rc représentent les groupements (CH₂)₀₋₃-Ar ou (CH₂)₀₋₃-Alk, Ar et Alk étant tels que définis précédemment et pouvant être substitués ou non substitués, ainsi que les sels d'addition avec les acides ou les bases.

Dans un septième groupe préféré, l'invention a pour objet les composés de formule générale (I) telle que définie précédemment, dans laquelle G est un groupement G4 de formule -NH-C(=NH)-NHRc, Rc étant tel que défini précédemment, ainsi que les sels d'addition avec les acides ou les bases.

Dans un huitième groupe préféré, l'invention a pour objet les composés de formule générale (I) telle que définie précédemment, dans laquelle G est un groupement G4 de formule NH-C(=NH)-NH₂, ainsi que les sels d'addition avec les acides ou les bases.

Dans un dixième groupe préféré, l'invention a pour objet les composés de formule générale (I) telle que définie précédemment, dans laquelle G est le groupement 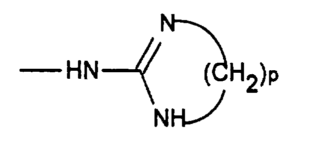 p étant un entier égal à 2, 3 ou 4, ainsi que les sels d'addition avec les acides ou les bases.

Dans un onzième groupe préféré, l'invention a pour objet les composés de formule (I) telle que définie précédemment dont les noms suivent:
- S-[4-[(4,5-dihydro-1H-imidazol-2-yl)hydrazono]-9,10-diméthoxy-1,2,3,4,5,6-hexahydro-8-benz[e]azulenyl]-N-[(phénylméthoxy)carbonyl]-DL-homocysteine
- Acide 4-[[4-[(4,5-dihydro-1H-imidazol-2-yl)hydrazono]-9,10-diméthoxy-1,2,3,4,5,6-hexahydro-8-benz[e] azulenyl]sulfinyl]-2-[[(phénylméthoxy)carbonyl]amino] butanoïque
- Acide 4-[[4-[(4,5-dihydro-1H-imidazol-2-yl) hydrazono]-9,10-diméthoxy-1,2,3,4,5,6-hexahydro-8-benz[e]azulenyl] sulfonyl]-2-[[(phénylméthoxy)carbonyl]amino] butanoïque

L'invention a également pour objet un procédé de préparation des composés de formule générale (I) comprenant les étapes suivantes :
a) action en présence d'une base, d'un composé de formule (F1) : 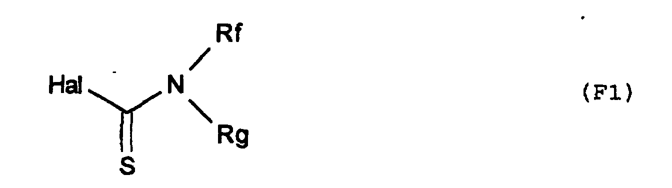 Hal représentant un atome de chlore ou de brome, Rf et Rg représentant un radical alkyle renfermant de 1 à 4 atomes de carbone,
   sur un composé de formule (II) : 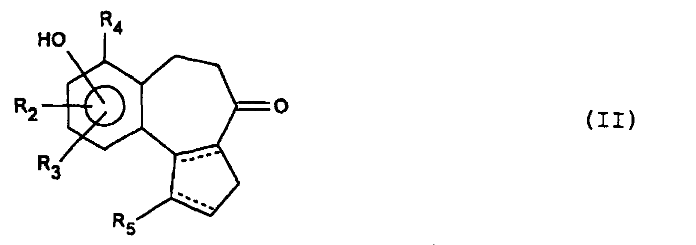 dans laquelle R₂, R₃, R₄ et R₅ sont tels que décrits précédemment à l'exception de la valeur hydroxyle,
   afin d'obtenir le composé de formule (IIIa) : 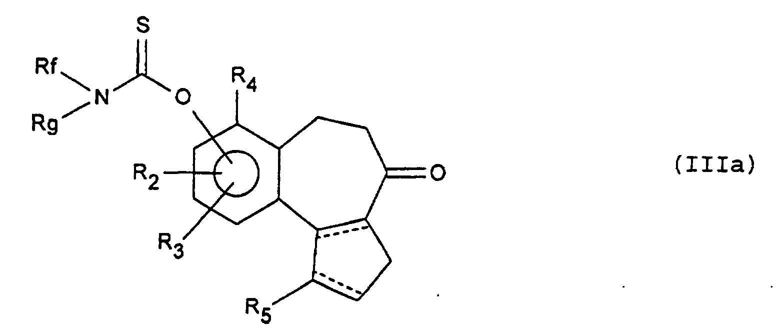
b) pyrolyse du composé de formule (IIIa) afin d'obtenir le composé de formule (IIIb) : 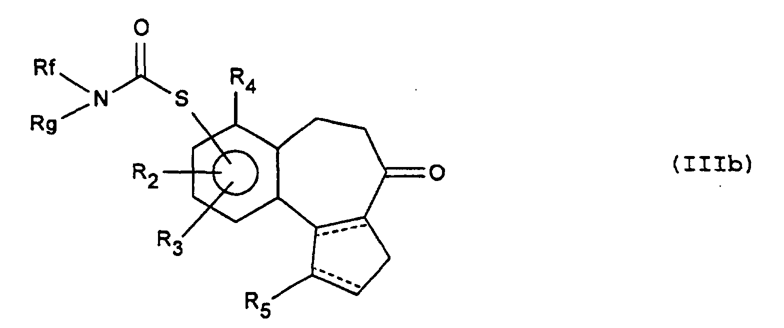
c) hydrolyse en milieu basique du composé de formule (IIIb) puis neutralisation afin d'obtenir le thiol de formule (IIIc) : 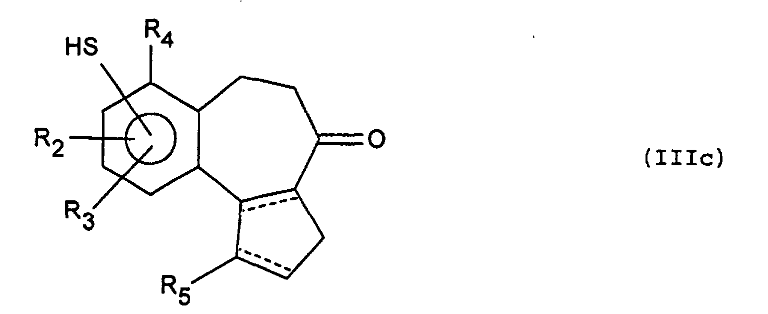
d) action sur le composé de formule (IIIc) d'un composé de formule (F2) :

   Hal-[A]-[B]-COR₂ (F2)

   Hal, [A], [B] et R₆ étant tels que définis précédemment,
   [B] pouvant également représenter le groupement -CH-NHP, P étant un groupement protecteur de la fonction amine, afin d'obtenir un composé de formule (IIId) : 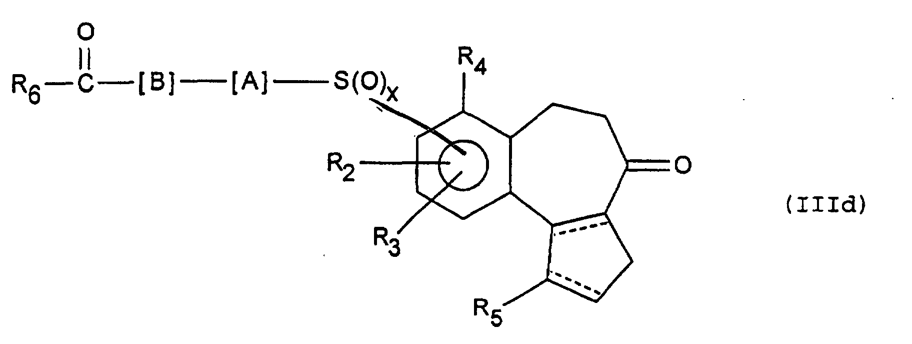 dans laquelle x est égal à 0,
e) action sur le composé de formule (IIId) d'un composé de formule (F3) :

   H₂N-G (F3)

   dans laquelle G est tel que défini précédemment afin d'obtenir un composé de formule (IV), correspondant aux composés de formule (I) avec x = 0 : 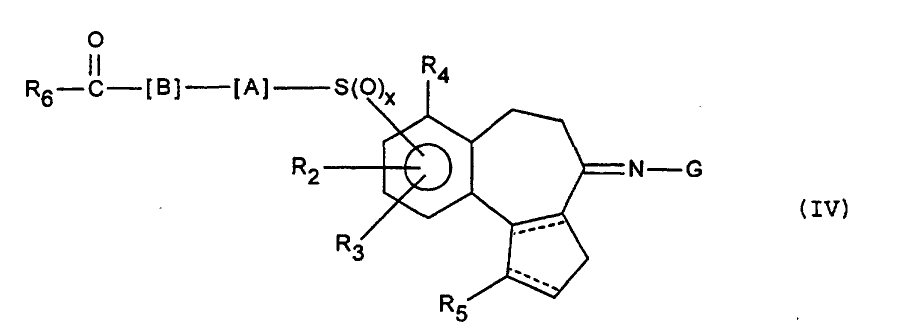
f) composé de formule (IV) que l'on soumet le cas échéant dans un ordre approprié :
   - à l'action d'un agent oxydant du soufre, afin d'obtenir les composés de formule (IV) avec x = 1 ou 2,
   - à l'action d'une base ou d'un acide afin de cliver l'ester -COR₆ et d'obtenir l'acide correspondant,
   - à l'action d'un agent de déalkylation,
   - à l'action d'un agent de déprotection de la fonction NH-P en bêta de CO-R₆ lorsque [B] représente le groupe CH-NHP,
   - à la formation du groupement NH-SO₂R_{c}, NH-CO₂R_{c}, NHCOR_{c}, NH-SO₂-NH-R_{c}, NH-CO-NHR_{c} à partir de l'amine correspondante en bêta de COR₆,
   - à l'action d'un acide ou d'une base afin d'obtenir les sels correspondants ou à l'action d'un agent d'estérification afin d'obtenir les esters correspondants.

L'action de l'halogénure de dialkylthiocarbamate de formule (F1) s'effectue de préférence selon les conditions décrites par M.S. Newman J. Org. Chem. 31, 3980 (1966).

La réaction de pyrolyse s'effectue de préférence à 260°C environ.

L'hydrolyse en milieu basique des composés de formule (IIIb) s'effectue de préférence en présence de soude 2N.

L'action du composé de formule Hal-[A]-[B]-COR₆ (F2) est effectuée de préférence en présence d'une base minérale telle que le carbonate de potassium ou le carbonate de sodium en présence d'un solvant dipolaire aprotique tel que le diméthylformamide. Hal est de préférence un atome de chlore ou de brome.

L'action de NH₂-G (F3) s'effectue, soit sans solvant, soit dans un solvant alcoolique tel que l'éthanol, l'isopropanol ou le butanol. Le synthon NH₂-G est éventuellement utilisé sous la forme d'un sel tel que le chlorhydrate ou le bromhydrate.

La réaction de saponification de la fonction ester s'effectue par exemple par action d'une base alcaline telle que la soude ou la potasse dans le tétrahydrofuranne ou un alcool inférieur tel que le méthanol ou l'éthanol. On peut également cliver l'ester en milieu acide selon les méthodes connues de l'homme du métier.

La réaction d'oxydation du soufre s'effectue de préférence selon la méthode décrite par W.C. Stevens dans Synthesis (1997) 764. La quantité de monoperoxyphtalate de magnésium utilisé sera déterminante pour l'obtention du sulfoxyde ou de la sulfone (0,6 ml d'oxydant pour obtenir le sulfoxy, 1,2 ml d'oxydant si l'on veut obtenir la sulfone).

La réaction d'oxydation aura lieu de préférence sur le composé de formule (IIId), c'est-à-dire avant l'introduction du groupement G par action de (F3) (étape e).

La réaction de déalkylation permettant d'accéder aux produits de formule (I) avec R₂, R₃, R₄ ou R₅ représentant. des hydroxyles s'effectue en présence de chlorure d'aluminium ou de tribromure de bore.

La fonctionnalisation de NH₂ en alpha de COR₆, [B] représentant CH(NH₂) ou CH(NH₂.Hcl), s'effectue selon les méthodes classiques connues en chimie organique.

La formation de NHSO₂R_{c} à partir de l'amine correspondante s'effectue de préférence par action de R_{c}SO₂Hal en présence d'une base par exemple la triéthylamine.

La formation de NHCO₂R_{c} à partir de l'amine correspondante s'effectue de préférence par action de R_{c}OH selon la méthode décrite dans J. Org. Chem., 61, 3929-3934 après avoir fait agir au préalable le triphosgène en présence de bicarbonate de sodium afin d'obtenir intermédiairement l'isocyanate.

Les réactions de salification peuvent être effectuées dans des conditions usuelles. On opère par exemple, pour salifier le groupement terminal CO₂H de R₁, en présence d'un sel de sodium tel que le carbonate de sodium ou le carbonate acide de sodium ou de potassium.

De même, la salification de l'amine ou de l'amino-guanidine que peuvent représenter G, par un acide, est réalisée dans les conditions usuelles. On opère par exemple avec l'acide chlorhydrique, par exemple en solution éthérée.

L'estérification éventuelle des produits est effectuée dans les conditions classiques connues de l'homme du métier.

On opère en général en faisant réagir l'acide de formule (I) ou un dérivé fonctionnel avec un réactif capable d'introduire le groupe ester dont une liste non exhaustive figure ci-dessus dans la définition de R₆.

Les produits de formule générale (F1), (F2) ou (F3) sont connus ou préparés selon les méthodes connues de l'homme du métier.

On peut également inverser l'ordre de greffage des différents réactifs, à savoir on soumet le composé de formule (II) à l'action d''un composé de formule F3 afin d'obtenir intermédiairement le produit de formule (IIIe) : 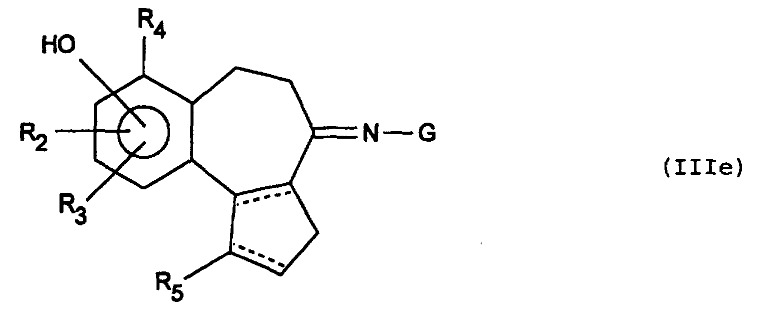 qui est mis en oeuvre ensuite dans les réactions telles que décrites aux étapes a), b), c), d) et le cas échéant f), afin d'obtenir les composés de formule (I).

Dans ce cas, il faudra le cas échéant, prévoir une protection du groupement G du produit de formule (IIIe) puis une déprotection selon les méthodes connues de l'homme du métier (T.W. GREENE Protective Groups in Organic Synthesis. John Wiley and Sons Inc. 1991).

La réaction de déprotection du groupement NH-P en bêta de CO-R₆, [B] représentant le groupe CH-NHP, s'effectue également selon les méthodes connues de l'homme du métier, notamment lorsque P représente le groupe CO₂tBu, par une réaction de décarboxylation telle que par exemple par action de l'acide chlorhydrique.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. L'ostéoporose est caractérisée par une perte sèche de cette matrice osseuse. Un ostéoclaste mature activé résorbe l'os après adhésion à la matrice osseuse via la sécrétion d'enzymes protéolytiques, et de protons à l'intérieur de la zone d'adhésion, aboutissant à des dépressions ou des creusements de la surface de l'os qui apparaissent au moment où l'ostéoclaste se détache de l'os.

Les composés de formule (I) ainsi que leurs sels d'addition pharmaceutiquement acceptables présentent d'intéressantes propriétés pharmacologiques. Ces composés inhibent la résorption osseuse qui est médiée via les ostéoclastes.

Les composés de l'invention sont ainsi utiles dans le traitement de maladies provoquées par la perte de la matrice osseuse, notamment, l'ostéoporose, l'hypercalcémie de malignité, l'ostéopénie due aux métastases osseuses, les parodontites, l'hyperparathyroidisme, les érosions periarticulaires dans l'arthrite rhumatoïde, la maladie de paget, l'ostéopénie induite par l'immobilisation, les traitements par les glucocorticoïdes ou les déficiences d'hormones sexuelles mâles ou femelles.

Ils peuvent également être utilisés pour le traitement des désordres inflammatoires, cancéreux et cardiovasculaires incluant l'athérosclérose, la resténose.

Ils peuvent enfin être utilisés comme inhibiteurs de l'angiogenèse et donc dans le traitement des tumeurs, par inhibition de leur néovascularisation, des rétinopathies diabétiques et des néphropathies.

Des études récentes ont montré que la fixation de l'ostéoclaste à l'os est médié par des récepteurs : les intégrines.

Les intégrines sont une superfamille de récepteurs médiant les processus d'adhésion cellule/cellule et plus particulièrement cellule/matrice, incluant notamment α2bβ3 comme récepteur des plaquettes sanguines (fibrinogène) et αvβ3 comme récepteur de la vitronectine, des sialoprotéines osseuses comme l'ostéopontine et la thrombospondine.

Ces récepteurs qui sont des hétérodimères protéiques composés de deux sous unités α et β, possèdent des sites de fixation d'ions divalents comme le Ca²⁺ notamment et un site de reconnaissance de leur ligand prédéfini par la qualité de leurs sous unités.

Le récepteur αvβ3 est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés selon l'invention.

Les récepteurs αvβ3 exprimés au niveau de la membrane des ostéoclastes sont à la base du processus d'adhésion/résorption, contribuent à l'organisation du cytosquelette cellulaire, et sont impliqués dans l'ostéoporose (Ross et al., J. Biol. Chem., 1987, 262, 7703).

Les récepteurs αvβ3 exprimés au niveau des cellules du muscle lisse de l'aorte, stimulent leur migration vers la neointima, ce qui entraîne la formation de l'athérosclérose et la survenue de resténose post-angioplastique (Brown et al, cardiovascular Res. (1994), 28, 1815).

Les cellules endothéliales sécrètent des facteurs de croissance qui sont mitogènes pour l'endothélium et peuvent contribuer à la formation de nouveaux vaisseaux sanguins (Angiogenèse). La stimulation angiogénique provoque la formation de nouveaux vaisseaux sanguins.

Les antagonistes de l'intégrine αvβ3 peuvent ainsi entraîner une régression des tumeurs cancéreuses en induisant l'apoptose des vaisseaux sanguins angiogéniques. (Brook et al. Cell (1994) 79, 1157).

Les ligands naturels de l'intégrine αvβ3 contiennent tous le motif RGD (Arg-Gly-Asp). Les peptides contenant ce motif RGD ainsi que des anticorps anti αvβ3 sont connus pour leur capacité d'inhibition de la résorption de la dentine, d'empêchement de l'adhésion des ostéoclastes sur les matrices minéralisées (Horton et al. Exp. Cell. Res. (1991), 195, 368) .

Le peptide Echistatine isolé du venin de serpent contenant également un motif RGD est décrit comme inhibiteur de l'adhésion des ostéoclastes à l'os, et est donc un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1441).

Les composés de formule (I) ainsi que leurs sels d'addition et leurs esters pharmaceutiquement acceptables peuvent posséder notamment une affinité vis-à-vis du récepteur de la vitronectine αvβ3 ou vis-à-vis d'autres intégrines ayant pour ligand la vitronectine (αvβ1, αvβ5, α2bβ3) en inhibant la liaison à leur ligand naturel.

Cette propriété rend ainsi les composés de l'invention. utilisables pour la prévention ou le traitement de maladies dont la pathologie sous-jacente est provoquée par les ligands ou les cellules qui interagissent avec le récepteur de la vitronectine.

Ces composés peuvent posséder également une activité vis-à-vis d'autres intégrines qui interagissent avec leur ligand via la séquence tripeptidique RGD, leur conférant des propriétés pharmacologiques utilisables pour traiter les pathologies associées à ces récepteurs.

Cette activité vis-à-vis des intégrines rend ainsi les composés de l'invention utilisable dans le traitement de nombreuses maladies telles que celles mentionnées plus haut ou dans la revue de Dermot Cox DN&P 8(4) Mai 1995, 197-205 dont le contenu est intégré dans la présente demande.

L'invention a donc pour objet les composés de formule (I) à titre de médicaments, ainsi que leurs sels d'addition ou leurs esters pharmaceutiquement acceptables.

Parmi les médicaments de l'invention, on peut citer particulièrement les composés décrits dans la partie expérimentale.

Parmi ces produits, l'invention a plus particulièrement pour objet, à titre de médicaments, les composés de formule (I) listés précédemment.

La posologie varie en fonction de l'affection à traiter et de la voie d'administration : elle peut varier par exemple de 1 mg à 1000 mg par jour chez l'adulte par voie orale.

L'invention s'étend aux compositions pharmaceutiques renfermant comme principe actif au moins un médicament tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, de nanosphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II), dans laquelle le radical hydroxy est en position 10, R₂ en position 8 et R₃ en position 9, représentent un groupement O-Me, R₄ et R₅ sont des atomes d'hydrogène, sont préparés selon la méthode décrite dans la demande de brevet européen n° 0729933 et dans la demande de brevet international WO 97/34865 (préparation 2).

Les deux autres isomères de position peuvent être préparés de la manière suivante :

On soumet un composé de formule (IIA) : 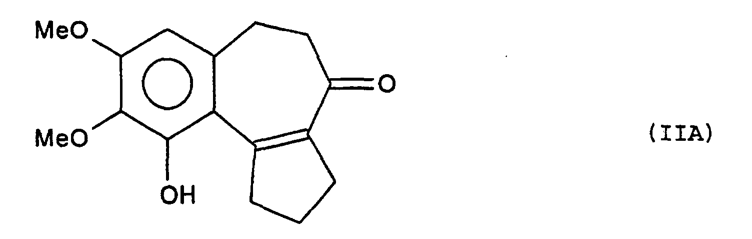 à l'action d'un réactif de déalkylation, afin d'obtenir le composé de formule (IIB) : 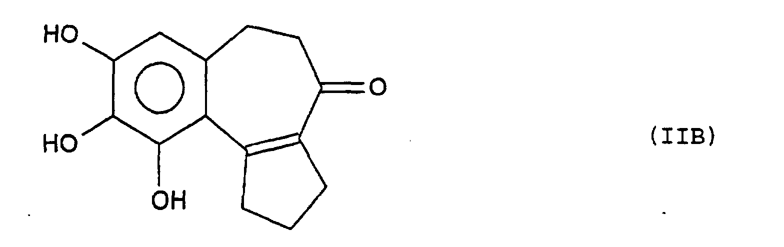 composé de formule (IIB) que l'on soumet :
**soit** à l'action d'un réactif de protection des diols en milieu basique, afin d'obtenir sélectivement le produit de formule (IIC) : 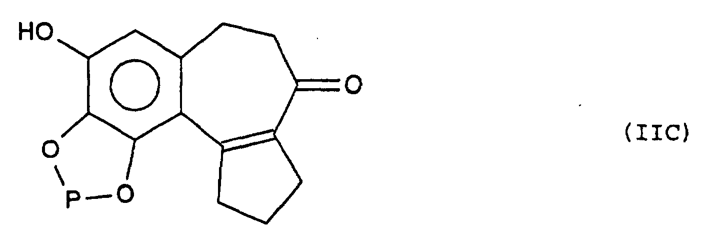 dans laquelle P représente le reste d'un réactif de protection des diols,
   que l'on soumet successivement à l'action d'un réactif de protection du phénol, d'un réactif de déprotection des diols, d'un agent d'alkylation puis d'un agent de déprotection du phénol afin d'obtenir le composé de formule (IID) correspondant au produit de formule (II) trisubstitué avec OH en position 8 : 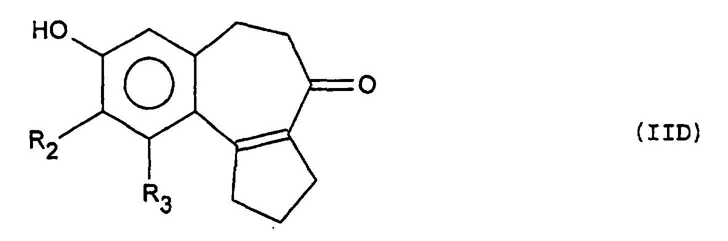
**soit** à l'action successivement d'un agent de protection du phénol, d'un agent d'alkylation puis d'un agent de déprotection afin d'obtenir le composé de formule (IIE) correspondant au produit de formule (II) trisubstitué avec OH en position 9

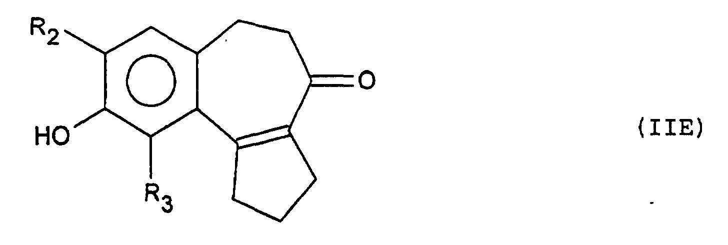

Par réactif de déalkylation, on entend de préférence des agents tels que le tribromure de bore ou le chlorure d'aluminium.

Le réactif de protection des diols que l'on fait réagir sur les produits de formule (IIB) peut être un dérivé du bore tel que l'acide borique, un borate de trialkyle, par exemple de triméthyle ou de triéthyle, ou encore le borax.

Par agent de protection du phénol, on entend notamment un halogénure tel que le chlorure ou le bromure de mésyle ou de tosyle ou encore un dérivé benzylé tel que le tosylate ou le mésylate de benzyle.

Par réactif de déprotection des diols, on entend notamment un acide fort tel que l'acide chlorhydrique, l'acide sulfurique ou bien l'acide paratoluène sulfonique ou encore un oxydant, par exemple l'eau oxygénée, dans le cas d'une protection par un dérivé du bore.

Par agent d'alkylation, on entend tout agent classique connu de l'homme du métier pour alkyler les phénols. On peut citer, par exemple un halogénure d'alkyle tel que le chlorure de méthyle ou d'éthyle, un sulfate d'alkyle tel que le sulfate de méthyle ou d'éthyle, ou encore le diazométhane.

Par agent de déprotection, on entend une base telle que la soude, la potasse ou encore le carbonate de sodium ou de potassium.

Les produits de formule (II) monosubstitués, dans lesquels R₂, R₃, R₄ et R₅ représentent un atome d'hydrogène, sont préparés selon une méthode analogue à celle décrite dans la demande de brevet européen n° 0729933 :
(i) On soumet un composé de formule (a) : 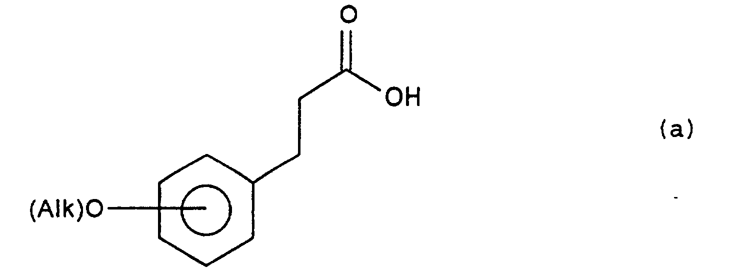 dans laquelle O-(Alk) est en position méta ou para du groupement alkylcarboxylique, (Alk) étant tel que défini précédemment, à l'action d'un agent d'halogénation pour obtenir l'halogénure d'acyle correspondant,
(ii) que l'on soumet à l'action d'un réactif de formule (b) : 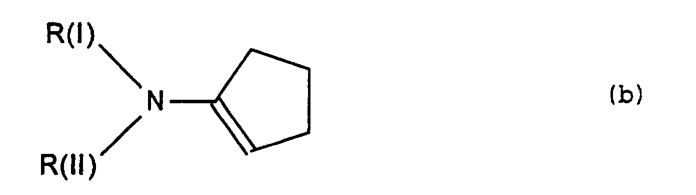 dans laquelle R(I) et R(II), identiques ou différents représentent un groupement alkyle renfermant de 1 à 6 atomes de carbone, ou R(I) et R(II) ensemble avec l'atome d'azote auquel ils sont liés, représentent un hétérocycle à 5 ou 6 chaînons, saturé ou insaturé, renfermant éventuellement un autre hétéroatome choisi parmi O et N,
   pour obtenir un composé de formule (c) : 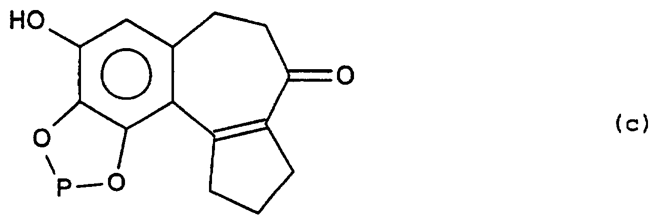
(iii) que l'on soumet à l'action d'un agent d'halogénation pour obtenir un composé de formule (d) : 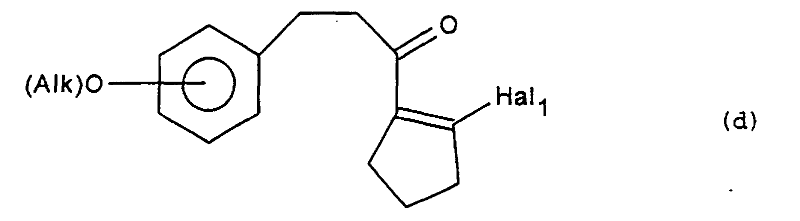 dans laquelle Hal₁ représente un atome d'halogène,
(iv) que l'on soumet à l'action d'un acide de Lewis, pour obtenir un composé de formule (e) : 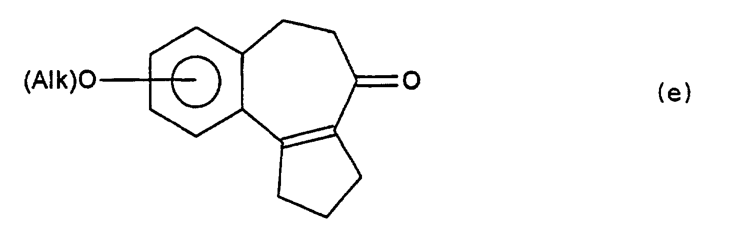
(v) que l'on soumet à un réactif de déalkylation afin d'obtenir le produit de formule (IIF) correspondant au produit de formule (II) monosubstitué attendu :

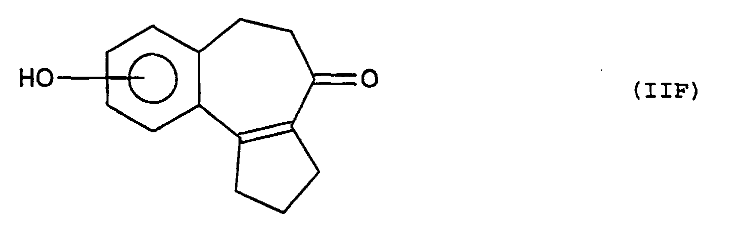

Les produits de formule (II) disubstitués, dans laquelle R₂ représente O-Me, R₃, R₄ et R₅ sont des atomes d'hydrogène et OH et R₂ étant en position 8, 9 ou 10, sont préparés selon la méthode telle que décrite ci-dessus à partir du composé de formule (a') : 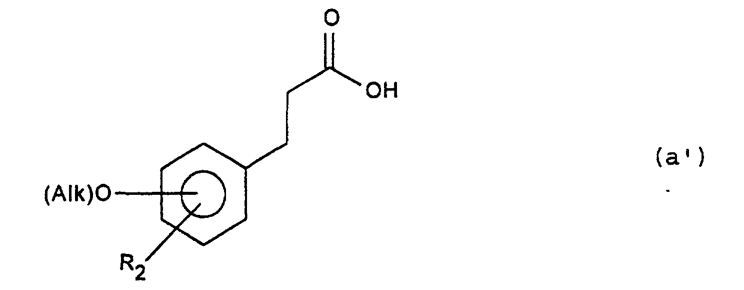 dans laquelle O-(Alk) et R₂ sont en position méta ou para de la chaîne alkyle carboxylique, R₂ étant un groupement O-Me, successivement aux réactions (i), (ii), (iii), (iv) et (v) et on obtient les produits de formule (IIG) correspondant aux produits de formule (II) bisubstitués attendus :

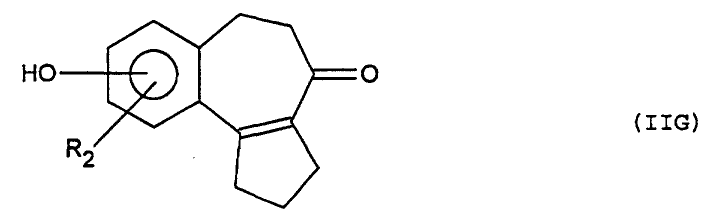

L'agent d'halogénation que l'on fait agir sur le composé de formule (a) ou (a') est par exemple le chlorure de thionyle, le chlorure d'oxalyle ou tout autre agent connu de l'homme du métier pour préparer un halogénure d'acide.

Le réactif de formule (b) est préparé au départ de la cyclopentanone et d'une amine secondaire, par exemple la diéthylamine, la pipéridine, la pipérazine ou, de préférence, la morpholine. On opère en présence d'un catalyseur acide fort, par exemple l'acide paratoluène sulfonique.

L'action de l'énamine de formule (b) sur l'halogénure d'acide est réalisée de préférence en présence d'une amine tertiaire telle que la triéthylamine ou la pyridine.

L'agent d'halogénation que l'on fait réagir sur le composé de formule (c), ou son équivalent disubstitué de formule (c'), peut être par exemple le chlorure de thionyle, le phosgène, l'oxychlorure de phosphore ou, de préférence, le chlorure d'oxalyle.

L'acide de Lewis utilisé pour cycliser le composé de formule (d), ou son équivalent disubstitué de formule (d') est par exemple le chlorure d'aluminium, le tétrachlorure de titane, ou de préférence le chlorure ferrique, ou le tétrachlorure d'étain. La réaction, comme celles qui précèdent, peut être conduite, par exemple, dans un solvant halogéné tel que le chlorure de méthylène, le chloroforme ou le dichloroéthane.

Le réactif de déalkylation du composé de formule (e), ou son équivalent disubstitué de formule (e') afin d'obtenir les phénols correspondant est de préférence le chlorure d'aluminium ou le tribromure de bore.

Les produits de formule (II) dans laquelle R₅ est un atome d'hydrogène et dans laquelle il y a une double liaison en position 1-2 sont préparés selon les méthodes connues de l'homme du métier et notamment selon la méthode décrite dans la demande de brevet européen n° 0729933, c'est-à-dire par déshydratation ou désalcoxylation en milieu acide anhydre.

Les produits de formule (II) dans laquelle la jonction entre le cycle à 5 et le cycle à 7 est saturée sont préparés selon les méthodes classiques d'hydrogénation notamment en présence de palladium sur charbon de la double liaison correspondante.

La réaction d'hydrogénation s'effectue de préférence sur les composés de formule (IIA), (IID), (IIE), (IIF) ou (IIG).

L'invention a également pour objet, à titre de produits intermédiaires, les produits de formule (IIIa), (IIIb), (IIIc) et (IIId).

Les exemples suivants illustrent l'invention sans toutefois la limiter.

### EXEMPLE 1 : S-[4-[(4,5-dihydro-1H-imidazol-2-yl)hydrazono]-9,10-diméthoxy-1,2,3,4,5,6-hexahydro-8-benz[e]azulenyl]-N-[(phénylméthoxy)carbonyl]-DL-homocysteine

### Stade A : diméthyl carbamothioate de O-(9,10-diméthoxy-1,2,3,4,5,6-hexahydro-4-oxo-8-benz[e]azulenyle Introduction de la liaison O-CS-NMe₂ en position 8

On ajoute à 5°C sous atmosphère inerte à 2,74 g de 9,10-diméthoxy-8-hydroxy-2,3,5,6-tétrahydro-benz[e]azulen-4(1H)-one obtenu comme indiqué à la préparation 3 de la demande de brevet WO 97/34865 dans 3 ml de diméthylformamide, 45 mg d'hydrure de sodium, puis 144 mg de chlorure de diméthylthiocarbamoyl dans 1 ml de diméthylformamide et chauffe 16 heures à 80°C.

Après avoir versé dans une solution de bicarbonate et extrait à l'acétate d'éthyle et évaporé sous pression réduite on obtient 400 mg de produit brut que l'on purifie par chromatographie en éluant avec le mélange dichlorométhane/acétone 17-3. On obtient 176 mg de produit pur attendu.
F = 134-135°C.

| RMN (CDCl₃) | |
|---|---|
| 1,88 (m) 2H | CH₂ en position 2 |
| 2,63 (m) 2H | =C-CH₂ |
| 2,75 (m) 2,86 (sl) | |
| 3,83 (s) | =C-N-Me |
| 3,48 (s) | |
| 3,83 (s) | =C-OCH₃ |
| 3,88 (s) | |
| 6,76 (s) | Ph-H (H en position 7) |

### Stade B : diméthyl carbamothioate de S-(9,10-diméthoxy-1,2,3,4,5,6-hexahydro-4-oxo-8-benz[e]azulenyle Pyrolyse

On chauffe 635 mg du produit obtenu au stade précédent à 260°C pendant 5 minutes. On obtient 475 mg de produit attendu. F = 136-137°C.

| RMN (CHCl₃) | |
|---|---|
| 1,88 (m) | CH₂ en position 2 |
| 4,66 (m) 2,76 (m) | =C-CH₂ |
| 4,85 (m) 3,10 (m) | |
| 3,04 (sl) 3,14 (sl) | CONMe₂ |
| 3,82 (s) 3,86 (s) | =C-OCH₃ |
| 7,16 (s) | Ph-H (H en position 7) |

### Stade C : 9,10-diméthoxy-8-thiol-2,3,5,6-tétrahydro-benz[e] azulen-4(1H)-one

### Hydrolyse : Obtention du thiol

On chauffe reflux pendant 7 h 30, 474 mg du produit obtenu au stade précédent, 12 ml de méthanol et 1 ml de soude 2N, rajoute 0,5 ml de soude 2N et chauffe au reflux pendant encore 30 minutes. Après avoir ramené le pH à 4,5 avec de l'acide chlorhydrique 2N on extrait, lave, évapore sous pression réduite jusqu'à obtention de 500 mg de produit brut attendu.

### Stade D : S-[9,10-diméthoxy-1,2,3,4,5,6-hexahydro-4-oxo-8-benz[e]azulenyl]-N-[(phénylméthoxy)carbonyl]-DL-homocysteinate d'éthyle

### Alkylation

On mélange sous atmosphère inerte 500 mg du produit obtenu au stade précédent, 4 ml de diméthylformamide, 400 mg de carbonate de potassium et 20 mg de 4-diméthylamino pyridine et 520 mg de 4-bromo-2-[[(phénylméthoxy)carbonyl] amino]butyrate d'éthyle et agite 1 nuit à température ambiante. On verse dans de l'eau, extrait à l'acétate d'éthyle, lave et évapore sous pression réduite jusqu'à obtention de 800 mg de produit brut que l'on purifie par chromatographie en éluant avec le mélange dichlorométhane/acétone 18-2. On obtient 564 mg de produit pur attendu.

| RMN (CDCl₃) | |
|---|---|
| 1,27 (t) 4,22 (q) | CO₂Et |
| 1,87 (m) | CH₂ en position 2 |
| 2,04 (m) 2,25 (m) | S-CH₂₋CH₂ de la chaîne |
| 2,63 (m) 2H | |
| 2,75 (m) 2H | |
| 2,81 (m) 2H | S-CH₂-CH₂, =C-CH₂ |
| 2,96 (m) 2H | |
| 3,04 (m) 2H | |
| 3,80 (s) 3,83 (s) | =C-OCH₃ |
| 4,51 (m) | CH₂-CH-NH-C(O)- |
| 5,46 (dl) | CH₂-CH-NH-C(O)- |
| 5,12 (s) | CO₂-CH2-Ph |
| 6,81 (s) | Ph-H, H en position 7 |
| 7,35 | 5H aromatique |

### Stade E : S-[4-[(4,5-dihydro-1H-imidazol-2-yl)hydrazono]-9,10-diméthoxy-1,2,3,4,5,6-hexahydro-8-benz[e]azulenyl]-N-[(phénylméthoxy)carbonyl]-DL-homocysteinate d'éthyle Introduction du groupement G

A 240 mg du produit obtenu au stade précèdent, on ajoute 2,5 mg d'isopropanol, 9 mg d'acide paratoluènesulfonique monohydraté, 40 mg de bromhydrate de 2-hydrazino-2-imidazoline et agite 30 minutes au reflux. On évapore sous pression réduite jusqu'à obtention de 400 mg de produit brut que l'on purifie par chromatographie en éluant avec le mélange dichloroéthane/méthanol/hydroxyde d'ammonium 95/5/0,5. On obtient 266 mg (rf CH₂Cl₂/MeOH/NH₂OH = 0,25).

| RMN (CDCl₃) | |
|---|---|
| 1,26 (t) 4,20 (q) | CO₂Et |
| 1,88 (m) | CH₂ en position 2 |
| 4,03 (m) 2,20 (m) | S-CH₂₋CH₂ |
| 2,70 à 3,10 (m) 10H | S-CH₂ et =C-CH₂ |
| 3,59 (s) 4H | N-CH₂-CH₂-N |
| 3,74 (s) 3,82 (s) | CH₃O-C= |
| 4,49 (m,dd après échange) | CO-NH-CH- |
| 5,47 (dl mobile) | CO-NH-CH- |
| 5,12 (s) | CO₂-CH₂Ph |
| 6,76 (s) | Ph-H, H en position 7 |
| 7,35 (m) | 5H aromatique |

### Stade F : S-[4-[(4,5-dihydro-1H-imidazol-2-yl)hydrazono]-9,10-diméthoxy-1,2,3,4,5,6-hexahydro-8-benz[e]azulenyl]-N-[(phénylméthoxy)carbonyl]-DL-homocysteine

A 155 mg de produit obtenu au stade précédent, dans 3 ml de méthanol, on ajoute 0,5 ml de soude 2N et agite 1 h 30 à température ambiante. Après avoir ajusté le pH à 5-6, en ajoutant 0,5 à 0,6 ml d'acide chlorhydrique 2N, on évapore sous pression réduite et purifie par chromatographie le produit brut en éluant avec le mélange dichlorométhane/ méthanol/hydroxyde d'ammonium 85-15-1,5. On obtient 122 mg de produit pur attendu.

| RMN (CDCl₃) | |
|---|---|
| 1,75 à 2,10 (m) 4H | CH₂ en position 2, S-CH₂-CH₂ |
| 2,60 à 3,10 (m) 10H | S-CH₂ et CH₂-C= |
| 3,57 (sl) 4H | N-CH₂-CH₂-N |
| 3,69 (s) 6H | CH₃O-C= |
| 4,00 (m) | CH-NHCO |
| ∼ 7,18 (m) | CH-NHCO |
| 5,02 (AB) | CO-CH₂-CO₂ |
| 6,89 (s) | Ph-H, H en position 7 |
| 7,13 à 7,40 (m) | 5H aromatique |

### EXEMPLE 2 : Acide 4-[[4-[(4,5-dihydro-1H-imidazol-2-yl)hydrazono]-9,10-diméthoxy-1,2,3,4,5,6-hexahydro-8-benz[e]azulenyl]sulfinyl]-2-[[(phénylméthoxy)carbonyl]amino] butanoïque

### Stade A : 4-[[9,10-diméthoxy-1,2,3,4,5,6-hexahydro-4-oxo-8-benz[e]azulenyl]sulfinyl]-2-[[(phénylméthoxy)carbonyl]amino] butanoate d'éthyle

### Oxydation

Sous atmosphère inerte, on mélange 1 g de dioxyde de silice (0,06-0,04 m) et 0,5 ml d'eau, agite 20 minutes jusqu'à absorption de l'eau, puis ajoute 2 ml de dichlorométhane, 171 mg de monoperoxyphtalate de magnésium, 280 mg du produit obtenu au stade D de l'exemple 1 et encore 3,6 ml de dichlorométhane. Après 3 heures d'agitation à température ambiante, on essore le dioxyde de silice, lave, sèche et évapore sous pression réduite. On obtient 170 mg de produit attendu.

| IR (CHCl₃) | |
|---|---|
| =C-NH | 3426 cm⁻¹ |
| C=O | 1722, 1651 cm⁻¹ |
| C=C + | 1597, 1577, 1554, 1508 cm⁻¹ |

aromatique +
amide II
Absorption région S=O

### Stade B : Acide 4-[[4-[(4,5-dihydro-1H-imidazol-2-yl)hydrazono]-9,10-diméthoxy-1,2,3,4,5,6-hexahydro-8-benz[e]azulenyl]sulfinyl]-2-[[(phénylméthoxy)carbonyl]amino] butanoïque

### Introduction de G puis saponification

On opère comme aux stades E puis F de l'exemple 1 mais à partir de 160 mg du produit obtenu au stade précédent. On obtient 152 mg de produit pur attendu.

| RMN (CDCl₃) | |
|---|---|
| 1,65 à 2,30 (m) | CH₂ en position 2, S-CH₂-CH₂ |
| 2,60 à 3,20 (m) 11H | CH₂-C= |
| 3,57 (sl) 3,60 4H | N-CH₂-CH₂-N |
| 3,70 (s) 3,76 (s) | CH₃O-C= |
| 3,78 (s) 6H | |
| 3,7 à 4,1 | CH₂₋CH-NHCO |
| 4,95 à 5,20 (m) | CO₂-CH₂Ph |
| 7,10 à 7,40 (m) 8H | Ph-H, H en position 7, H aromatique |
| 7,7 large | H mobile. |

### EXEMPLE 3 : Acide 4-[[4-[(4,5-dihydro-1H-imidazol-2-yl) hydrazono]-9,10-diméthoxy-1,2,3,4,5,6-hexahydro-8-benz[e]azulenyl]sulfonyl]-2-[[(phénylméthoxy)carbonyl]amino] butanoïque

### Stade A : 4-[(9,10-diméthoxy-1,2,3,4,5,6-hexahydro-4-oxo-8-benz[e]azulenyl)sulfonyl]-2-[[(phénylméthoxy)carbonyl]amino] butanoate d'éthyle

On opère comme à l'exemple 2, stade A à partir de 1,10 mg du produit obtenu au stade D de l'exemple 1 et en utilisant 370 mg (1,2 ml) d'oxydant. On obtient 80 mg de produit pur attendu.

| IR (CHCl₃) | |
|---|---|
| NH | 3424 cm⁻¹ |
| C=O | 1723 cm⁻¹, 1655 cétone conjuguée |
| aromatique + amide II | 1600, 1578, 1508 cm⁻¹ |
| SO₂ | 1307, 1142 cm⁻¹ |

### Stade B : Acide 4-[[4-[(4,5-dihydro-1H-imidazol-2-yl) hydrazono]-9,10-diméthoxy-1,2,3,4,5,6-hexahydro-8-benz[e]azulenyl]sulfonyl]-2-[[(phénylméthoxy)carbonyl]amino] butanoïque

On opère comme au stade B de l'exemple 2 (introduction de G puis saponification) à partir de 74 mg de la sulfone obtenue au stade précédent. On obtient 76 mg de produit pur attendu.

| RMN (CDCl₃) | |
|---|---|
| 1,80 à 2,15 (m large) 4H | CH₂ en position 2, S-CH₂-CH₂ |
| 2,84 (m large) | CH₂-C= |
| 3,30 à 3,55 (m) | CH₂X |
| 3,60 (sl) 4H | N-CH₂-CH₂-N |
| 3,70 (s) 3,84 (s) | CH₃O-C= |
| 3,92 (m) | CH₂-CH-NHCO |
| 7,15 (m large mobile) | CH₂-CH-NHCO |
| 4,99 (AB) | CO₂CH₂Ph |
| 7,34 (m) | Phényle |
| 7,55 (s) | Ph-H, H en position 7 |
| 7,90 (ep) | H mobile. |

### EXEMPLE 4 : S-[4-[(2-imidazolidinylidène)hydrazono]-1,2,3,4,5,6-hexahydro-8-benz[e]azulenyl]-N-[(phénylméthoxy) carbonyl]-DL-homocysteine

### Stade A : diméthylcarbamothioate de O-[1,2,3,4,5,6-hexahydro-4-oxo-8-benz[e]azulenyle]

A 450 mg d'hydrure de sodium on ajoute goutte à goutte 2,14 g de 8-hydroxy-2,3,5,6-tétrahydro-benz[e]azulenate(1)one préparé comme indiqué à la préparation 7 de la demande de brevet WO 97/34865 en solution dans 15 ml de diméthylformamide. A la fin du dégagement d'hydrogène, on refroidit à 0°/+5°C le mélange et ajoute 1,65 g de chlorure de diméthylthiocarbamoyle puis chauffe 3 heures à 50°C ± 5°C et maintient 16 heures sous agitation à température ambiante. On verse ensuite 200 ml d'une solution de bicarbonate de sodium au demi, extrait à l'acétate d'éthyle, lave à l'eau salée, sèche, élimine les solvants sous pression réduite, chromatographie le résidu sur silice (éluant CH₂Cl₂-AcOEt 95-5) et recueille 2,45 g de produit attendu. F = 116°-118°C.

| IR (CHCl₃) | |
|---|---|
| C=O | 1640 cm⁻¹ |
| Système conjugué ) + Aromatiques ) | 1604, 1594, 1570, 1537, 1496 cm⁻¹ |

### Stade B : diméthylcarbamothioate de S-[1,2,3,4,5,6-hexahydro-4-oxo-8-benz[e]azulenyle]

### Réarrangement thermique

On chauffe à 260°C environ, sous agitation pendant 6 minutes, 2,4 g de produit obtenu au stade précédent. On refroidit, ajoute du dichlorométhane, chromatographie sur silice (éluant CH₂Cl2-AcOEt 95/5) et obtient 1,97 g de produit attendu.

| IR (CHCl₃) | |
|---|---|
| C=O | 1654 cm⁻¹ |
| Aromatiques | 1597, 1588, 1550, 1485 cm⁻¹ |

### Stade C : 8-mercapto-2,3,5,6-tétrahydro-4-(1H) benz[e]azulenone

On introduit 100 mg de produit obtenu au stade précédent dans 1 ml d'éthylène glycol, ajoute 0,1 ml d'une solution comprenant 870 mg de potasse dans 1 ml d'eau, chauffe 30 minutes au reflux, laisse revenir à température ambiante, ajoute de l'acide chlorhydrique 2N jusqu'à l'obtention du pH : 3-4, extrait au dichlorométhane, lave à l'eau salée, sèche, élimine les solvants sous pression réduite et obtient 56 mg de produit attendu après chromatographie sur silice (éluant CH₂Cl₂-AcOEt 97/3).

| IR (CHCl₃) | |
|---|---|
| C=O | 1639 cm⁻¹ |
| C=C + Aromatiques | 1595, 1549, 1489 cm⁻¹ |

### Stade D : S-[1,2,3,4,5,6-hexahydro-4-oxo-8-benz[e]azulenyle]-N-[(phénylméthoxy)carbonyl]-DL-homocystéinate d'éthyle

A 738 mg de produit obtenu comme au stade C, dans 10 ml de diméthylformamide, on ajoute 1,28 g de carbonate de potassium, 64 mg de 4-diméthylaminopyridine et 1,67 g de 4-bromo-2-[[(phénylméthoxy)carbonyl]amino]butanoate d'éthyle préparé de manière analogue à celle de l'ester méthylique décrit à la préparation 8 de la demande de brevet international WO 97/34865.

On agite 40 minutes, verse le milieu réactionnel dans 60 ml d'eau, extrait à l'éther, lave à l'eau salée, évapore le solvant sous pression réduite, chromatographie le résidu sur silice (éluant CH₂Cl₂-acétone 98/2) et obtient 1,46 g de produit attendu.

| IR (CHCl₃) | |
|---|---|
| NH | 3434 cm⁻¹ |
| C=O | 1722 (max. complexe) 1639 cm⁻¹ |
| Aromatiques ) + Amide II ) | 1592, 1545, 1507 cm⁻¹ |

### Stade E : bromhydrate de S-[4-[2-imidazolidinylidène) hydrazono]-1,2,3,4,5,6-hexahydro-8-benz[e]azulenyle]-N-[(phénylméthoxy)carbonyl]-DL-homocystéinate d'éthyle

On chauffe au reflux pendant 13 heures, 260 mg de produit obtenu au stade D dans 2,5 ml d'isopropanol en présence de 11 mg d'acide paratoluène sulfonique et 115 mg de bromhydrate de 2-hydrazino 2-imidazoline. On laisse revenir à température ambiante, dissout l'insoluble dans le méthanol au reflux, glace, essore les cristaux et recueille 299 mg de produit attendu. F = 226-227°C.

### Stade F : S-[4-[(2-imidazolidinylidène)hydrazono]-1,2,3,4,5,6-hexahydro-8-benz[e]azulenyl]-N-[(phénylméthoxy) carbonyl]-DL-homocysteine

On agite au reflux pendant 45 minutes, 250 mg du produit obtenu au stade E dans 20 ml de méthanol en présence de 1 ml de soude 2N. On laisse revenir à température ambiante, acidifie jusqu'à pH 5-6 avec de l'acide chlorhydrique 2N, évapore les solvants sous pression réduite, reprend le résidu dans 100 ml de mélange de dichlorométhane/méthanol 50/50, évapore de nouveau les solvants, chromatographie le résidu sur silice (éluant CH₂Cl₂-MeOH-NH₄OH 80/20/4) et récupère 181 mg de produit attendu. F ≃ 160°C.

| RMN : (DMSO) | |
|---|---|
| 1,83 (m) (2H) | CH₂ en 9 |
| 1,94 (m) (2H) | CH₂ chaîne |
| 2,71 (sl) (4H) ) | les =C-CH₂ |
| 2,87 (sl) (4H) ) | et Φ-S-CH₂ |
| 3,00 (m) (2H) ) | |
| 3,55 (sl) (4H) | les =N-CH₂ |
| 3,99 (q) (1H) | =C-CH-N-C= |
| 5,02 (sl) (2H) | COO-CH₂-Φ |
| ≅ 7,13 à 7,38 (m) (8H) ) ) | Φ-C et H aromatiques |

### EXEMPLE 5 : chlorhydrate de l'acide 4-[[4-[(2-imidazolidinylidène)hydrazono]-1,2,3,4,5,6-hexahydro-8-benz[e] azulenyl]sulfonyl]-2-[[(phénylméthoxy)carbonyl]amino]-butanoïque

### Stade A : 4-[[1,2,3,4,5,6-hexahydro-4-oxo-8-benz[e]azulenyle] sulfonyl]-2-[[(phénylméthoxy)carbonyl]amino]-butanoate d'éthyle

On ajoute goutte à goutte 2,5 ml d'eau à 5 g de dioxyde de silice, maintient sous agitation pendant 20 minutes, ajoute 1,05 g de monoperoxyphtalate de magnésium et 25 ml de dichlorométhane, agite 20 minutes, ajoute 0,31 g du composé obtenu au stade D de l'exemple 4 dans 5 ml de dichlorométhane, agite 1 heure, filtre et rince au dichlorométhane, lave le milieu réactionnel avec une solution de métabisulfite de sodium puis de chlorure de sodium, sèche, évapore les solvants, chromatographie le résidu sur silice (éluant CH₂Cl₂/acétone 95/5) et obtient 302 mg de produit attendu.

| IR (CHCl₃) | |
|---|---|
| NH | 3423 cm⁻¹ |
| C=O | 1722 - 1650 cm⁻¹ |
| Aromatique ) + Amide II ) | 1601, 1589, 1507 cm⁻¹ |
| SO₂ | 1304, 1149 cm⁻¹ |

### Stade B : bromhydrate de 4-[[4-[(2-imidazolidinylidène)hydrazono]-1,2,3,4,5,6-hexahydro-8-benz[e] azulenyl]sulfonyl]-2-[[(phénylméthoxy)carbonyl]amino]-butanoate d'éthyle

On opère comme au stade E de l'exemple 4 en utilisant au départ 290 mg de produit obtenu au stade A, 3 ml d'isopropanol, 110 mg de bromhydrate de 2-hydrazine 2-imidazoline et 10 mg d'acide paratoluène sulfonique. On chromatographie le produit obtenu sur silice (éluant CH₂Cl₂-MeOH-NH₄OH 95/5/0,5) et obtient 194 mg de produit attendu. F = 209-210°C.

| RMN : (DMSO) | |
|---|---|
| 1,14 (t) ) | COOEt |
| 4,08 (q) ) | |
| ≅ 1,90 (m) | CH₂ en 9 |
| ≅ 2,05 (ml) | CH₂ central |
| 2,73 (l) (2H) ) | les =C-CH₂ |
| 2,90 à 3,00 (6H) ) | |
| 3,72 (sl) | les =N-CH₂ |
| 4,17 (m) (1H) | =C-CH-N-C= |
| 5,00 [AB] | COO-CH₂-Φ |
| ≅ 7,34 (l) | Φ-C |
| 7,60 (d,J=8) (1H) ) | =CH aromatiques |
| 7,75 à 7,85 (3H) | et =C-NH |
| 11,06 (sl) | H mobiles |

### Stade C : chlorhydrate de l'acide 4-[[4-[(2-imidazolidinylidène)hydrazono]-1,2,3,4,5,6-hexahydro-8-benz[e] azulenyl]sulfonyl]-2-[[(phénylméthoxy)carbonyl]amino]-butanoïque

On opère comme au stade F de l'exemple 4 en utilisant au départ 248 mg de produit obtenu au stade B ci-dessus et 1 ml de soude 2N dans 20 ml de méthanol.

Après ajustage à pH : 5-6, on concentre au demi, essore, rince à l'eau puis au méthanol glacé, puis à l'éther, sèche à 40°C sous pression réduite et obtient 227 mg de produit brut que l'on reprend dans 3 ml d'acide chlorhydrique N, dissout la gomme formée dans le méthanol, évapore le solvant sous pression réduite, rince à l'eau, sèche, et récupère 208 mg de produit attendu.

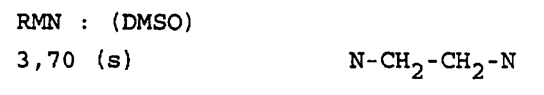

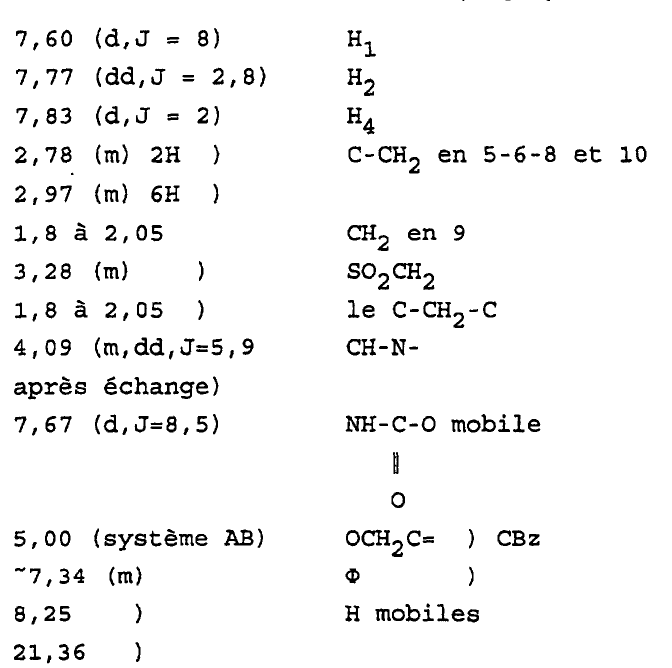

### EXEMPLE 6 : acide 4-[[4-[(2-imidazolidinylidène)hydrazono]-1,2,3,4,5,6-hexahydro-8-benz[e]azulenyl]sulfinyl]-2-[[(phénylméthoxy)carbonyl]amino]-butanoïque

### Stade A : 4-[[1,2,3,4,5,6-hexahydro-4-oxo-8-benz[e]azulenyle] sulfinyl]-2-[[(phénylméthoxy)carbonyl]amino]-butanoate d'éthyle

On opère comme à l'exemple 5 stade A, en utilisant au départ 1,25 g de dioxyde de silice, 0,63 ml d'eau, 210 mg de monoperoxyphtalate de magnésium, 6 ml de dichlorométhane et 300 mg du composé obtenu comme à l'exemple 4 stade D, dans 3 ml de dichlorométhane. On obtient 217 mg de produit attendu.

| IR (CHCl₃) | |
|---|---|
| NH | 3425 cm⁻¹ |
| C=O | 1721 - 1647 cm⁻¹ |
| C=C + Aromatique + Amide II | 1599, 1588, 1508 cm⁻¹ |
| S->O | 1044 cm⁻¹ |

### Stade B : 4-[[4-[4-[(2-imidazolidinylidène)hydrazono]-1,2,3,4,5,6-hexahydro-8-benz[e]azulenyl]sulfinyl]-2-[[(phénylméthoxy)carbonyl]amino]-butanoate d'éthyle

On opère comme à l'exemple 5 stade B en utilisant au départ 210 mg du produit obtenu au stade A, 2,2 ml d'isopropanol, 88 mg de bromhydrate de 2-hydrazine 2-imidazoline et 8 mg d'acide paratoluène sulfonique. Après chromatographie sur silice (éluant CH₂Cl₂-MeOH-NH₄OH 95/5/0,5), on obtient 225 mg de produit attendu.

| IR (CHCl₃) | |
|---|---|
| NH | 3450 cm⁻¹ (max) |
| C=O | 1721 cm⁻¹ (max) |
| C=N C=C + | 1664 (ep, f), 1623 (max, F), 1544, |
| Aromatique + Amide II | 1506, 1488 cm⁻¹ |
| S->O | 1044 cm⁻¹ |

### Stade C : acide 4-[[4-[(2-imidazolidinylidène)hydrazono]-1,2,3,4,5,6-hexahydro-8-benz[e]azulenyl)sulfinyl]-2-[[(phénylméthoxy)carbonyl]amino]-butanoïque

On opère comme au stade F de l'exemple 4, en utilisant 225 mg du produit obtenu au stade B, 8 ml de méthanol et 1 ml de soude 2N en chauffant 8 heures au reflux. On recueille 177 mg de produit attendu. F = 191-192°C.

| RMN : (DMSO) | |
|---|---|
| 1,60 à 2,20 (m) 4H | 2 CH₂ centraux |
| 2,65 à 3,10 (m) ∼11H | les CH₂-O |
| ∼ 3,57 (s large) ∼4H | N-CH₂-CH₂-N |
| 3,96 (m) | CO-NH-CH-CH₂ |
| 5,00 (s) 5,01 (s) | CO₂CH₂Φ |
| 7,15 à 7,50 (m) ∼8H + éther éthylique | aromatique |
| (CH₂SO supposé masqué). | |

### Compositions pharmaceutiques

On a préparé des comprimés répondant à la formule suivante :
- produit de l'exemple 1 50 mg
- Excipient (talc, amidon, stéarate de
   magnésium) QS pour un comprimé terminé à 120 mg

### Etude pharmacologique des produits de l'invention

### 1 - Etude par les produits de l'invention du déplacement de la liaison : Vitronectine/récepteur Vitronectine (αᵥβ₃)

### Protocole :

Des plaques 96 puits MaxiSorp sont coatées une nuit à 4°C, avec 100 µl de Vitronectine humaine (cf Yatohgo et al. Cell., Structure and fraction 13 : 281-292 (1988)) à 2 µg/ml, (Dilution en tampon de coating).

Le lendemain, les puits sont vidés et les ligands (Vitronectine) sont ensuite fixés (voir tampon de fixation) pendant 1H à température ambiante sous agitation douce.

Les puits sont lavés six fois (voir tampon de lavage), puis on ajoute par puits et dans cet ordre :
- 40 µl de tampon d'incubation,
- 10 µl de la dilution du produit à tester,
(les produits sont dilués dans un mélange 50/50 de DMSO-H₂O)
- 50 µl de récepteur αᵥβ₃ humain (cf Pytela et al. Methods Enzymol (1987) 144:475) (dilution en tampon d'incubation, à adapter suivant le lot de récepteur et selon le ligand).

Le ligand, le récepteur αᵥβ₃ humain et les produits à étudier sont incubés pendant 3 heures à température ambiante sous agitation douce.

Les puits sont à nouveau lavés six fois, puis incubés pendant 2 heures à température ambiante sous agitation douce, en présence de 100 µl d'anticorps 4B12-HRP, anti-récepteur couplé à une peroxydase (l'anticorps 4B12-HRP est dilué en tampon d'incubation. La dilution est à adapter suivant le lot de récepteur).

Les puits sont ensuite lavés six fois avant la mesure de liaison ligand-récepteur faite par l'intermédiaire d'un kit révélateur de peroxydase (TMB Microwell Peroxidase Substrate System Kirkegaard : Réf. cat. 50-76-00).

Ce kit contient un flacon A de substrat (3,3',5,5'-tétraméthylbenzidine à 0,4 g/l) et un flacon B (H₂O₂ à 0,02 % en tampon Citrate/Acide citrique). Extemporanément, un volume de A est mélangé à un volume de B, puis le mélange réactionnel est distribué à raison de 100 µl/puits. La réaction enzymatique se développe en 12' pour Vitronectine/αᵥβ₃, puis son évolution est stoppée par l'addition de 100 µl d'acide phosphorique 1M.
La densité optique est mesurée à 450 nm.

### Tampons :

- tampon de coating : Carbonate 0,05 M, NaOH pH 9,6
- tampon de fixation : PBS contenant 0,5 % de BSA (pH 7,4)
- tampon de lavage : PBS contenant 0,05 % de Tween 20 (pH 7,4)
- tampon d'incubation :
   . 50 mM TRIS pH 7,4
   . 0,5 % BSA
   . 0,05 % Tween 20
   . 1 mM MnCl₂
   . 50 µM CaCl₂
   . 50 µM MgCl₂
   . 100 mM NaCl.

### Expression des résultats :

On trace la courbe suivante : le pourcentage de liaison de la vitronectine humaine en fonction du logarithme de la concentration de chaque produit testé.

Pour chaque produit on détermine l'IC₅₀ suivant la formule suivante :

IC₅₀ = (BO + Bmin)/2

BO = Maximum de liaison en l'absence de tout produit
Bmin = Minimum de liaison en présence de la concentration la plus élevée de produit.

### RESULTATS :

| Exemples | Test de compétition binding Vn/VR ((αᵥβ₃) IC₅₀ en µM |
|---|---|
| EX. 1 | 0,040 |
| EX. 4 | 0,030 |
| EX. 5 | 0,033 |
| EX. 6 | 0,230 |

## Revendications

1. Composés de formule générale (I) : 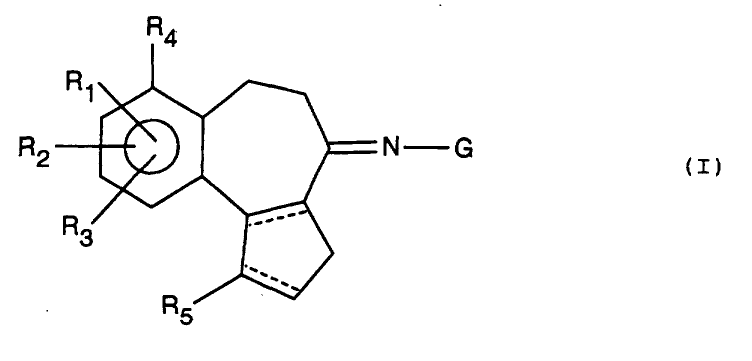 dans laquelle R₁ représente un groupement -S(O)ₓ-[A]-[B]-COR₆, x étant égal à 0, 1 ou 2, -[A]- représentant un radical bivalent alkylène, alkénylène ou alkynylène, linéaire ou ramifié, comportant de 1 à 6 atomes de carbone,
[B] représentant un radical phényle, un radical CH(Z), ou une simple liaison,
(Z) représente un atome d'hydrogène, un groupement (CH₂)₀₋₆-NRaRb, (CH₂)₀₋₆-NH-SO₂-Rc, (CH₂)₀₋₆-NH-CO₂-Rc, (CH₂)₀₋₆-NH-CO-Rc, (CH₂)₀₋₆-NH-SO₂-NH-Rc, (CH₂)₀₋₆-NH-CO-NH-Rc, (CH₂)₀₋₆-CO₂-Rc, (CH₂)₀₋₆-SO₂-Rc, (CH₂)₀₋₆-CO-Rc ou (CH₂)₀₋₆-Rc dans lesquels (CH₂)₀₋₆ est un radical bivalent alkylène comportant de 1 à 6 atomes de carbone, ou une simple liaison dans le cas de (CH₂)₀,
Ra, Rb et Rc représentent un atome d'hydrogène, un radical (CH₂)₀₋₃-Ar dans lequel Ar représente un groupement phényle ou un radical (CH₂)₀₋₃-Alk dans lequel Alk représente un radical alkyle, alkényle ou alkynyle, linéaire, ramifié ou cyclique, et comportant de 1 à 12 atomes de carbone, les radicaux Ar et Alk pouvant être non substitués ou substitués par
- halogène
- alkyle, alkényle, alkynyle renfermant de 1 à 12 atomes de carbone,
- oxo, cyano, nitro, formyl, carboxy et carboxyalkyle renfermant de 1 à 6 atomes de carbone, carboxamide,
- alkoxy renfermant de 1 à 12 atomes de carbone,
- alkylthio renfermant de 1 à 12 atomes de carbone,
- amino, alkylamino renfermant de 1 à 12 atomes de carbone, dialkylamino renfermant de 2 à 24 atomes de carbone,
- aminoalkyle renfermant de 1 à 12 atomes de carbone,
- dialkylaminoalkyle renfermant de 3 à 25 atomes de carbone,
- dialkylaminoalkyloxy renfermant de 3 à 25 atomes de carbone,
- hydroxyle éventuellement acylé renfermant de 1 à 12 atomes de carbone,
- acyle renfermant de 1 à 12 atomes de carbone ou benzoyle éventuellement substitués par un atome de chlore, d'iode ou de fluor,
- phényle, furyle, thiényle, pyridinyle ou benzyle éventuellement substitués par halogène, alkyle, alkoxy, alkylthio, aminoalkyle ou dialkylamino indiqués ci-dessus, ou encore, Ra et Rb représentent ensemble avec l'atome d'azote auquel ils sont liés un hétérocycle choisis parmi morpholine, pipéridine, pipérazine, pyrrolidine, pyrimidine, pyridine ou pyrazine,
- R₆ représente un groupement -OH, -OCH₃, -OCH₂CH₃, -O-(CH₂)₂-OH, -O-CH₂-CH(OH)-CH₂OH, -O-(CH₂)₂-NH₂, -O-(CH₂)₂-N-(CH₃)₂, -NH₂ ou -O-(CH₂)-phényle,
- R₂ et R₃ identiques ou différents représentent un atome d'hydrogène, un radical hydroxyle ou un radical O-Me,
- R₄ représente un atome d'hydrogène,
- R₅ représente un atome d'hydrogène,
- G représente,
**soit** un radical de formule G1 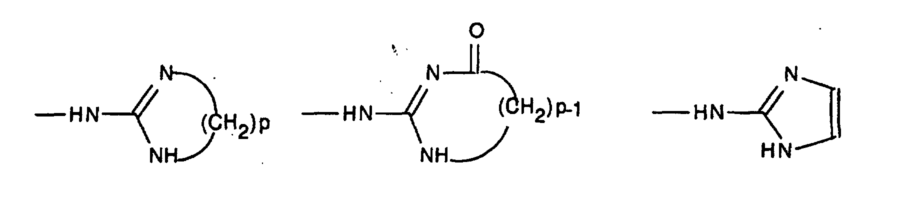 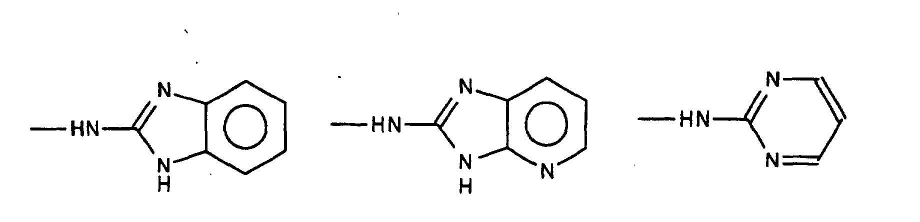 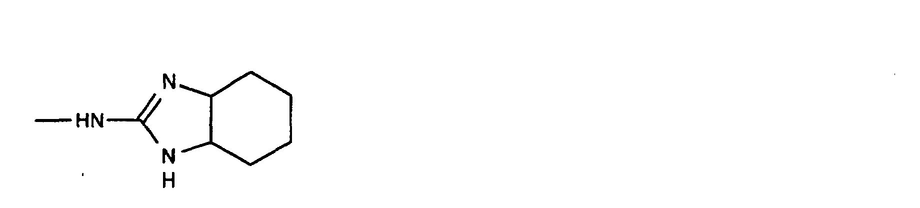 p étant un entier égal à 2, 3 ou 4,
- **soit** un radical NRaRb (radical G2), Ra et Rb étant tels que définis plus haut,
- **soit** un radical -NH-C(=X)-NHRc (radical G4), dans lequel X est un atome de soufre, d'oxygène ou NH et Rc est tel que défini précédemment,
- **soit** un radical -NH-SO₂Rc (radical G5), dans lequel Rc est tel que défini précédemment,
les traits en pointillés représentent une éventuelle seconde liaison, ainsi que les sels d'addition avec les acides ou les bases,
R₁, R₂ et R₃ peuvent être en position 8, 9 ou 10 du tricycle.

2. Composés de formule générale (I) telle que définie à la revendication 1, répondant à la formule générale (I') : 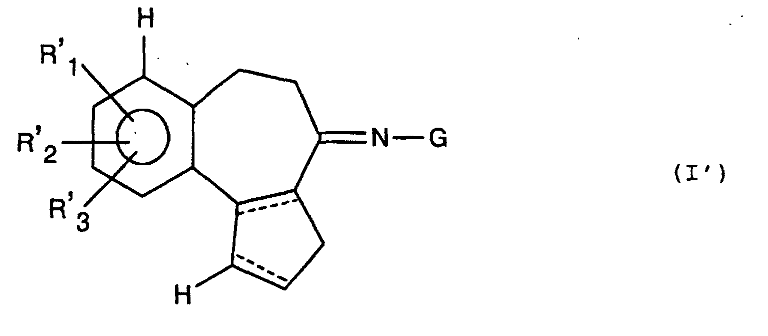 dans laquelle R'₁ représente un groupement -S(O)ₓ-[A']-[B']-COR'₆, x étant égal à 0, 1 ou 2, -[A']-représentant un radical bivalent alkylène, alkénylène ou alkynylène renfermant de 2 à 6 atomes de carbone, [B'] repré- sentant un radical CH(Z') ou une simple liaison,
(Z') représente un atome d'hydrogène, un groupement (CH₂)₀₋₆-NRaRb, (CH₂)₀₋₆-NH-SO₂-Rc, (CH₂)₀₋₆-NH-CO₂-Rc, (CH₂)₀₋₆-NH-CO-Rc, (CH₂)₀₋₆-NH-SO₂-NH-Rc,
(CH₂)₀₋₆-NH-CO-NH-Rc, (CH₂)₀₋₆-CO₂-Rc, (CH₂)₀₋₆-SO₂-Rc, (CH₂)₀₋₆-CO-Rc ou (CH₂)₀₋₆-Rc, Ra, Rb et Rc étant tels que définis à la revendication 1, R'₆ représente un groupement -OH, -OCH₃, -OCH₂CH₃, -O-(CH₂)₂-OH, -O-CH₂-CH(OH)-CH₂OH, -O-(CH₂)₂-NH₂, -O-(CH₂)₂-N-(CH₃)₂, -NH₂ ou -O-(CH₂)-phényle, R'₂ et R'₃ représentent un atome d'hydrogène ou un radical méthoxy, et G est tel que défini à la revendication 1, les traits en pointillés représentent une éventuelle seconde liaison, ainsi que les sels d'addition avec les acides ou les bases.

3. Composés de formule générale (I) telle que définie à la revendication 1 ou 2 dans laquelle R₆ représente un groupement -OH, -OCH₃, ainsi que les sels d'addition avec les acides ou les bases.

4. Composés de formule générale (I) telle que définie à la revendication 1, dans laquelle R₁ représente un groupement S(O)ₓ-(CH₂)₂-CH(Z')-COOH, x étant égal à 0, 1 ou 2, Z' étant tel que défini à la revendication 2, ainsi que les sels d'addition avec les acides ou les bases.

5. Composés de formule générale (I) telle que définie à la revendication 4, dans laquelle (Z') est le groupement (CH₂)₀₋₆-NH-CO₂-Rc ou (CH₂)₀₋₆-NH-Rb, Rb et Rc étant tels que définis à la revendication 1, ainsi que les sels d'addition avec les acides ou les bases.

6. Composés de formule générale (I) telle que définie à la revendication 5, dans laquelle (Z') est NHCO₂Rc ou NHRb, ainsi que les sels d'addition avec les acides ou les bases.

7. Composés de formule générale (I) telle que définie à la revendication 6, dans laquelle Rb et Rc sont les groupements (CH₂)₀₋₃-Ar ou (CH₂)₀₋₃-Alk, Ar et Alk étant tels que définis à la revendication 1, ainsi que les sels d'addition avec les acides ou les bases.

8. Composés de formule générale (I) telle que définie à l'une des revendications 1 à 7, dans laquelle G est un groupement G4 de formule générale -NH-C(=NH)-NHRc, Rc étant tel que défini à la revendication 1, ainsi que les sels d'addition avec les acides ou les bases.

9. Composés de formule générale (I) telle que définie à la revendication 8, dans laquelle Rc est un atome d'hydrogène, ainsi que les sels d'addition avec les acides ou les bases.

10. Composés de formule générale (I) telle que définie à la revendication 1, dans laquelle G est le groupement 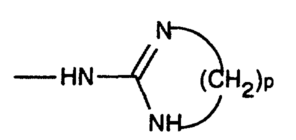 p étant un entier égal à 2, 3 ou 4, ainsi que les sels d'addition avec les acides ou les bases.

11. Composés de formule (I) telle que définie à la revendication 1 dont les noms suivent :
- S-[4-[(4,5-dihydro-1H-imidazol-2-yl)hydrazono]-9,10-diméthoxy-1,2,3,4,5,6-hexahydro-8-benz[e]azulenyl]-N-[(phénylméthoxy)carbonyl]-DL-homocysteine
- Acide 4-[[4-[(4,5-dihydro-1H-imidazol-2-yl)hydrazono]-9,10-diméthoxy-1,2,3,4,5,6-hexahydro-8-benz[e]azulenyl] sulfinyl]-2-[[(phénylméthoxy)carbonyl]amino] butanoïque
- Acide 4-[[4-[(4,5-dihydro-1H-imidazol-2-yl) hydrazono]-9,10-diméthoxy-1,2,3,4,5,6-hexahydro-8-benz[e]azulenyl] sulfonyl]-2-[[(phénylméthoxy)carbonyl]amino] butanoïque.

12. Procédé de préparation des composés de formule (I) telle que définie à la revendication 1, comprenant les étapes suivantes :
a) action en présence d'une base, d'un composé de formule (F1) : 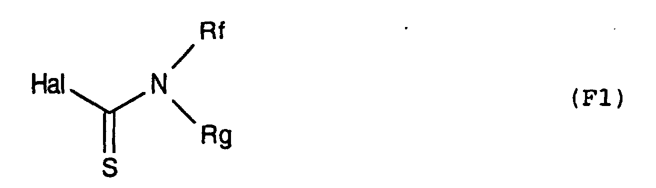 Hal représentant un atome de chlore ou de brome, Rf et Rg représentant un radical alkyle renfermant de 1 à 4 atomes de carbone,
sur un composé de formule (II) : 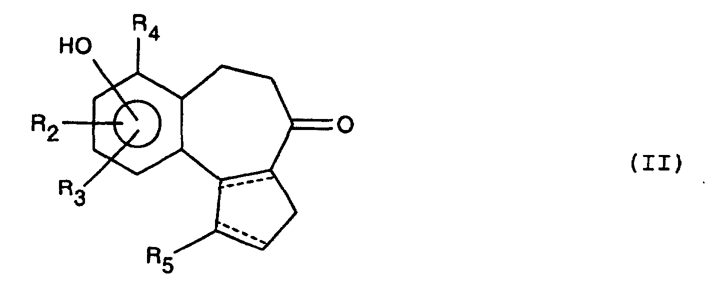 dans laquelle R₂, R₃, R₄ et R₅ sont tels que décrits à la revendication 1, à l'exception de la valeur hydroxyle,
afin d'obtenir le composé de formule (IIIa) : 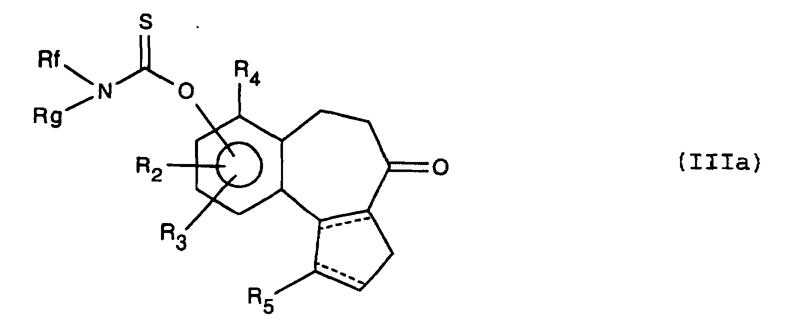
b) pyrolyse du composé de formule (IIIa) afin d'obtenir le composé de formule (IIIb) : 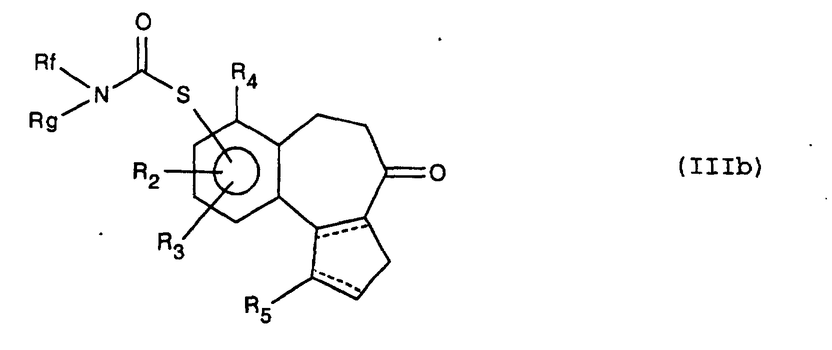
c) hydrolyse en milieu basique du composé de formule (IIIb) puis neutralisation afin d'obtenir le thiol de formule (IIIc) : 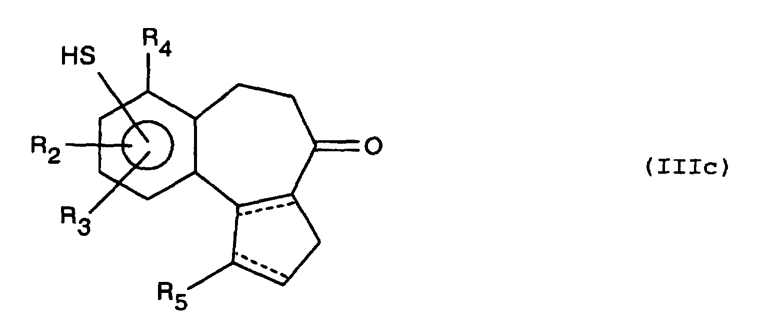
d) action sur le composé de formule (IIIc) d'un composé de formule (F2) :
Hal-[A]-[B]-COR₂ (F2)
Hal, [A], [B] et R₆ étant tels que définis à la revendication 1,
[B] pouvant également représenter le groupement -CH-NHP, P étant un groupement protecteur de la fonction amine, afin d'obtenir un composé de formule (IIId) : 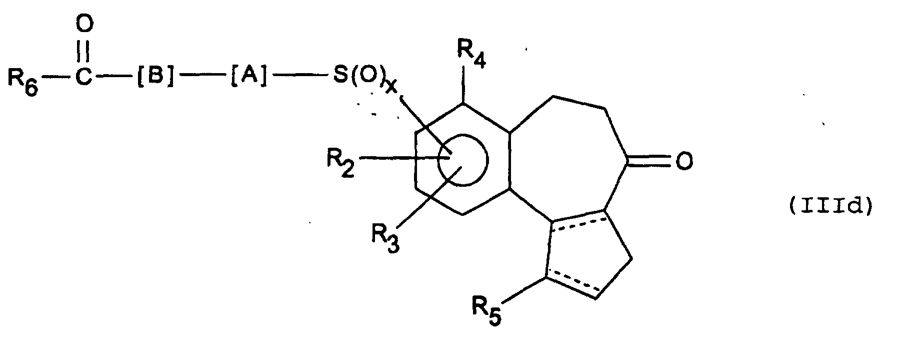 dans laquelle x est égal à 0,
e) action sur le composé de formule (IIId)d'un composé de formule (F3) :
H₂N-G (F3)
dans laquelle G est tel que défini à la revendication 1, afin d'obtenir un composé de formule (IV), correspondant aux composés de formule (I) avec x = 0 : 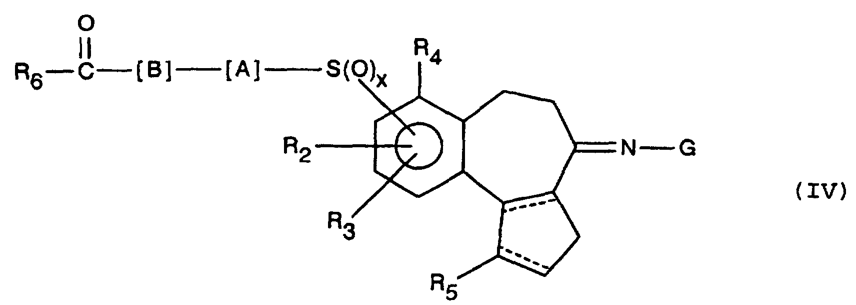
f) composé de formule (IV) que l'on soumet le cas échéant dans un ordre approprié :
- à l'action d'un agent oxydant du soufre, afin d'obtenir les composés de formule (IV) avec x = 1 ou 2,
- à l'action d'une base ou d'un acide afin de cliver l'ester -COR₆ et d'obtenir l'acide correspondant,
- à l'action d'un agent de déalkylation,
- à l'action d'un agent de déprotection de la fonction NH-P en bêta de CO-R₆ lorsque [B] représente le groupe CH-NHP,
- à la formation du groupement NH-SO₂R_{c}, NH-CO₂R_{c}, NHCOR_{c}, NH-SO₂-NH-R_{c}, NH-CO-NHR_{c} à partir de l'amine correspondante en bêta de COR₆,
- à l'action d'un acide ou d'une base afin d'obtenir les sels correspondants ou à l'action d'un agent d'estérification afin d'obtenir les esters correspondants.

13. Procédé de formation des composés de formule (I) telle que définie à la revendication 1, **caractérisé en ce que** l'on soumet préalablement un composé de formule (II) tel que défini à la revendication 12, à l'action d'un composé de formule (F3) tel que défini à la revendication 12 afin d'obtenir un composé de formule (IIIe) : 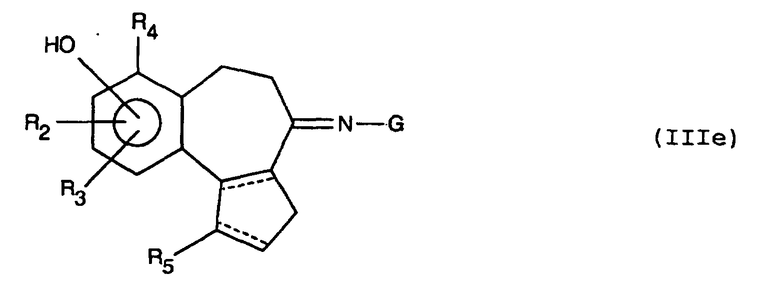 qui est mis en oeuvre ensuite dans les réactions telles que décrites aux étapes a), b), c), d) et le cas échéant f) telles que définies à la revendication 12.

14. A titre de médicament, les composés de formule (I) telle que définie à l'une quelconque des revendications 1 à 10, ainsi que leurs sels d'addition pharmaceutiquement acceptables.

15. A titre de médicament, les composés de formule (I) tels que définis à la revendication 11.

16. Les compositions pharmaceutiques renfermant comme principe actif au moins l'un des médicaments définis à la revendication 14 ou 15.

17. A titre de produits intermédiaires, les composés de formules générales (IIIa), (IIIb), (IIIc) et (IIId) telles que définies à la revendication 12.

## Claims

1. Compounds of general formula (I): 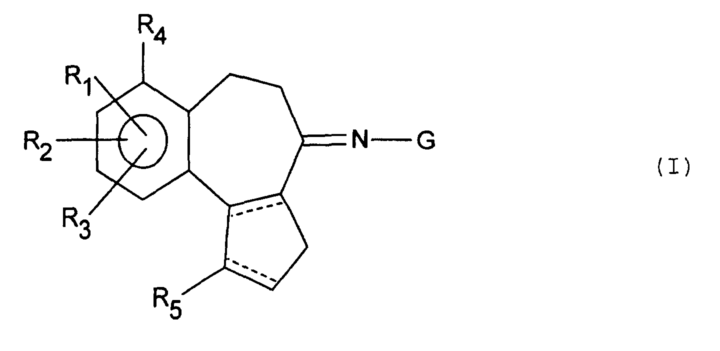 in which R₁ represents an
-S(O)ₓ-[A]-[B]-COR₆ group, x being equal to 0, 1 or 2, -[A]- representing a divalent linear or branched alkylene, alkenylene or alkynylene radical, comprising 1 to 6 carbon atoms,
[B] representing a phenyl radical, a CH(Z) radical, or a single bond,
(Z) represents a hydrogen atom, a (CH₂)₀₋₆-NRaRb, (CH₂)₀₋₆-NH-SO₂-Rc, (CH₂)₀₋₆-NH-CO₂-Rc, (CH₂)₀₋₆-NH-CO-Rc, (CH₂)₀₋₆-NH-SO₂-NH-Rc, (CH₂)₀₋₆-NH-CO-NH-Rc, (CH₂)₀₋₆-CO₂-Rc, (CH₂)₀₋₆-SO₂-Rc, (CH₂)₀₋₆-CO-Rc or (CH₂)₀₋₆-Rc group in which (CH₂)₀₋₆ is a divalent alkylene radical comprising 1 to 6 carbon atoms, or a single bond in the case of (CH₂)₀,
Ra, Rb and Rc represent a hydrogen atom, a (CH₂)₀₋₃-Ar radical in which Ar represents a phenyl group or a (CH₂)₀₋₃-Alk radical in which Alk represents a linear, branched or cyclic alkyl, alkenyl or alkynyl radical comprising 1 to 12 carbon atoms, the Ar and Alk radicals being able to be non substituted or substituted by
- halogen
- alkyl, alkenyl, alkynyl containing 1 to 12 carbon atoms,
- oxo, cyano, nitro, formyl, carboxy and carboxyalkyl containing 1 to 6 carbon atoms, caboxamide,
- alkoxy containing 1 to 12 carbon atoms,
- alkylthio containing 1 to 12 carbon atoms,
- amino, alkylamino containing 1 to 12 carbon atoms, dialkylamino containing 2 to 24 carbon atoms,
- aminoalkyl containing 1 to 12 carbon atoms,
- dialkylaminoalkyl containing 3 to 25 carbon atoms,
- dialkylaminoalkyloxy containing 3 to 25 carbon atoms,
- optionally acylated hydroxyl containing 1 to 12 carbon atoms,
- acyl containing 1 to 12 carbon atoms or benzoyl, optionally substituted by a chlorine, iodine or fluorine atom,
- phenyl, furyl, thienyl, pyridinyl or benzyl optionally substituted by halogen, alkyl, alkoxy, alkylthio, aminoalkyl or dialkylamino indicated above,
or also, Ra and Rb represent together with the nitrogen atom to which they are linked a heterocycle, chosen from morpholine, piperidine, piperazine, pyrrolidine, pyrimidine, pyridine or pyrazine,
- R₆ represents an -OH, -OCH₃, -OCH₂CH₃, -O-(CH₂)₂-OH, -O-CH₂-CH(OH)-CH₂OH, -O-(CH₂)₂-NH₂, -O-(CH₂)₂-N-(CH₃)₂, -NH₂ or
- O-(CH₂)-phenyl group
- R₂ and R₃ identical or different represent a hydrogen atom, a hydroxyl radical or an O-Me radical
- R₄ represents a hydrogen atom,
- R₅ represents a hydrogen atom,
- G represents,
**either** a radical of formula G1 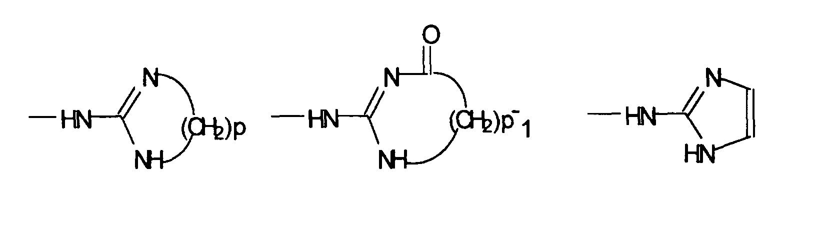 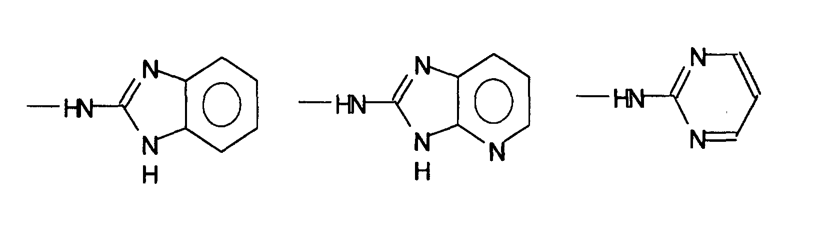 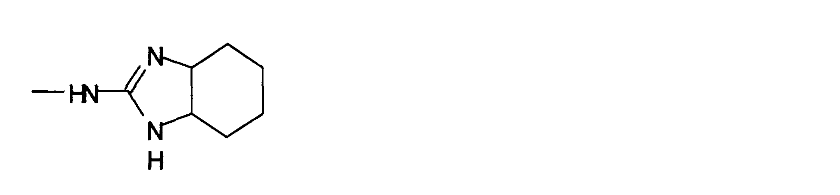 p being an integer equal to 2, 3 or 4
- **or** an NRaRb radical (G2 radical), Ra and Rb being as defined above,
- **or** an -NH-C(=X)-NHRc radical (G4 radical), in which X is a sulphur, oxygen atom or NH and Rc are as defined previously,
- **or** an -NH-SO₂Rc radical, (G5 radical), in which Rc is as defined previously,
the dotted lines represent an optional second bond, as well as the addition salts with acids or bases,
R₁, R₂ and R₃ can be in position 8, 9 or 10 of the tricycle.

2. Compounds of general formula (I) as defined in claim 1, corresponding to general formula (I'): 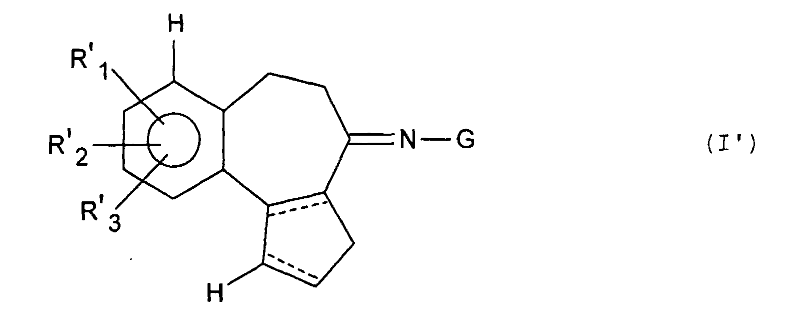 in which R'₁ represents an
-S(O)ₓ-[A']-[B']-COR'₆ group, x being equal to 0, 1 or 2,
-[A']- representing a divalent alkylene, alkenylene or alkynylene radical containing 2 to 6 carbon atoms, [B'] representing a CH(Z') radical or a single bond,
(Z') represents a hydrogen atom, a (CH₂)₀₋₆-NRaRb, (CH₂)₀₋₆-NH-SO₂-Rc, (CH₂)₀₋₆-NH-CO₂-Rc, (CH₂)₀₋₆-NH-CO-Rc, (CH₂)₀₋₆-NH-SO₂-NH-Rc, (CH₂)₀₋₆-NH-CO-NH-Rc, (CH₂)₀₋₆-CO₂-Rc, (CH₂)₀₋₆-SO₂-Rc, (CH₂)₀₋₆-CO-Rc or (CH₂)₀₋₆-Rc group, Ra, Rb and Rc being as defined in claim 1, R'₆ represents an -OH, -OCH₃, -OCH₂CH₃, -O-(CH₂)₂-OH, -O-CH₂-CH(OH)-CH₂OH, -O-(CH₂)₂-NH₂, -O-(CH₂)₂-N-(CH₃)₂, -NH₂ or -O-(CH₂)-phenyl group, R'₂ and R'₃ represent a hydrogen atom or a methoxy radical, and G is as defined in claim 1, the dotted lines represent an optional second bond, as well as the addition salts with acids or bases.

3. Compounds of general formula (I) as defined in claim 1 or 2 in which R₆ represents an -OH, -OCH₃ group, as well as the addition salts with acids or bases.

4. Compounds of general formula (I) as defined in claim 1, in which R₁ represents an S(O)ₓ-(CH₂)₂-OH(Z')-COOH group, x being equal to 0, 1 or 2, Z' being as defined in claim 2, as well as the addition salts with acids or bases.

5. Compounds of general formula (I) as defined in claim 4, in which (Z') is the (CH₂)₀₋₆-NH-CO₂-Rc or (CH₂)₀₋₆-NH-Rb group, Rb and Rc being as defined in claim 1, as well as the addition salts with acids or bases.

6. Compounds of general formula (I) as defined in claim 5, in which (Z') is NHCO₂Rc or NHRb, as well as the addition salts with acids or bases.

7. Compounds of general formula (I) as defined in claim 6, in which Rb and Rc are the (CH₂)₀₋₃-Ar or (CH₂)₀₋₃-Alk groups, Ar and Alk being as defined in claim 1, as well as the addition salts with acids or bases.

8. Compounds of general formula (I) as defined in one of claims 1 to 7, in which G is a G4 group of general formula -NH-C(=NH)-NHRc, Rc being as defined in claim 1, as well as the addition salts with acids or bases.

9. Compounds of general formula (I) as defined in claim 8, in which Rc is a hydrogen atom, as well as the addition salts with acids or bases.

10. Compounds of general formula (I) as defined in claim 1, in which G is the 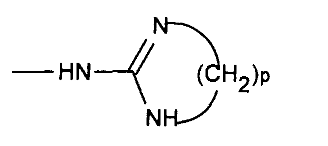 group, p being an integer equal to 2, 3 or 4, as well as the addition salts with acids or bases.

11. Compounds of formula (I) as defined in claim 1 the names of which follow:
- S-[4-[(4,5-dihydro-1H-imidazol-2-yl)hydrazono]-9,10-dimethoxy-1,2,3,4,5,6-hexahydro-8-benz[e]azulenyl]-N-[(phenylmethoxy)carbonyl]-DL-homocysteine
- 4-[[4-[(4,5-dihydro-1H-imidazol-2-yl)hydrazono]-9,10-dimethoxy-1,2,3,4,5,6-hexahydro-8-benz[e]azulenyl] sulphinyl]-2-[[(phenylmethoxy)carbonyl]amino] butanoic acid
- 4-[[4-[(4,5-dihydro-1H-imidazol-2-yl) hydrazono]-9,10-dimethoxy-1,2,3,4,5,6-hexahydro-8-benz[e]azulenyl] sulphonyl]-2-[[(phenylmethoxy)carbonyl]amino] butanoic acid.

12. Process for the preparation of the compounds of formula (I) as defined in claim 1, comprising the following stages:
a) action in the presence of a base, of a compound of formula (F1): 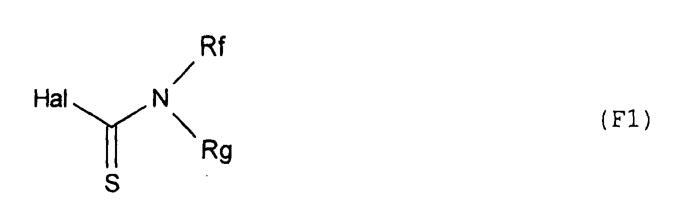 Hal representing a chlorine or bromine atom, Rf and Rg representing an alkyl radical containing 1 to 4 carbon atoms, on a compound of formula (II): 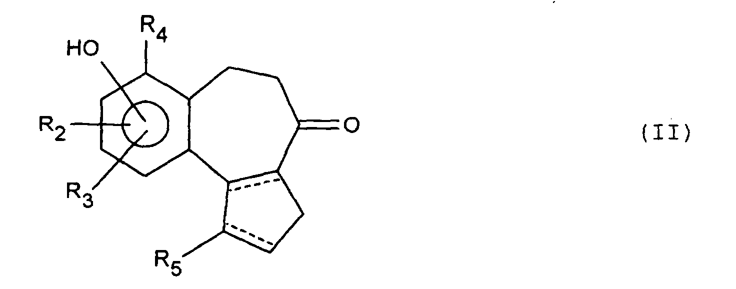 in which R₂, R₃, R₄ and R₅ are as described in claim 1, with the exception of the hydroxyl value,
in order to obtain the compound of formula (IIIa): 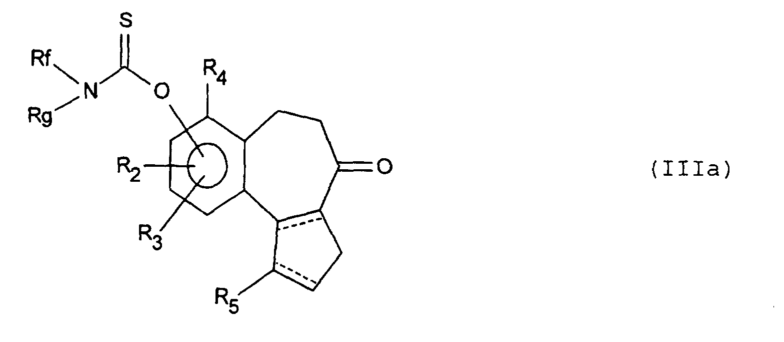
b) pyrolysis of the compound of formula (IIIa) in order to obtain the compound of formula (IIIb): 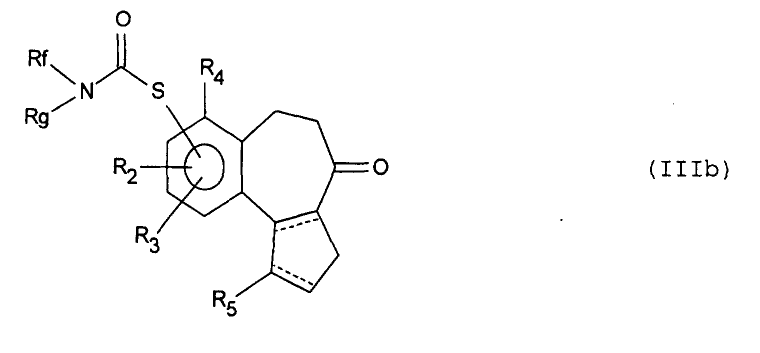
c) hydrolysis in basic medium of the compound of formula (IIIb) then neutralization in order to obtain the thiol of formula (IIIc) : 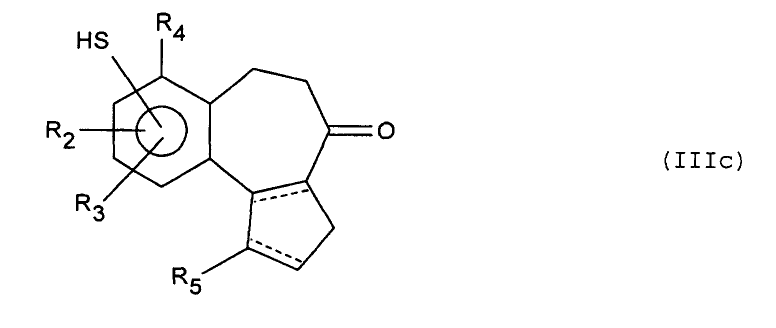
d) action on the compound of formula (IIIc) of a compound of formula (F2):
Hal-[A]-[B]-COR₂ (F2)
Hal, [A], [B] and R₆ being as defined in claim 1,
[B] can also represent the -CH-NHP group, P being a protective group of the amine function, in order to obtain a compound of formula (IIId): 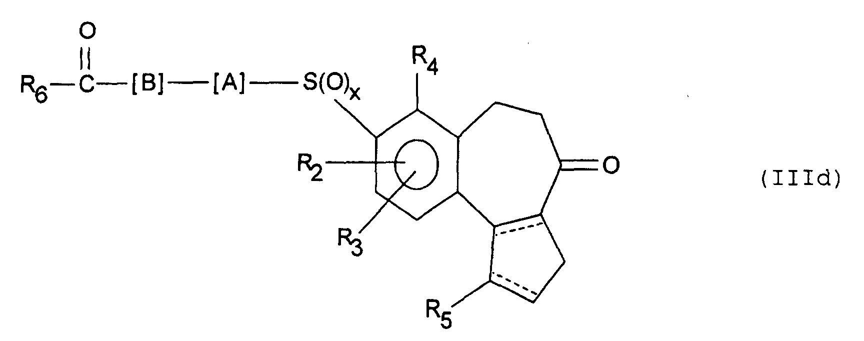 in which x is equal to 0,
e) action on the compound of formula (IIId) of a compound of formula (F3):
H₂N-G (F3
in which G is as defined in claim 1, in order to obtain a compound of formula (IV), corresponding to the compounds of formula (I) with x = 0: 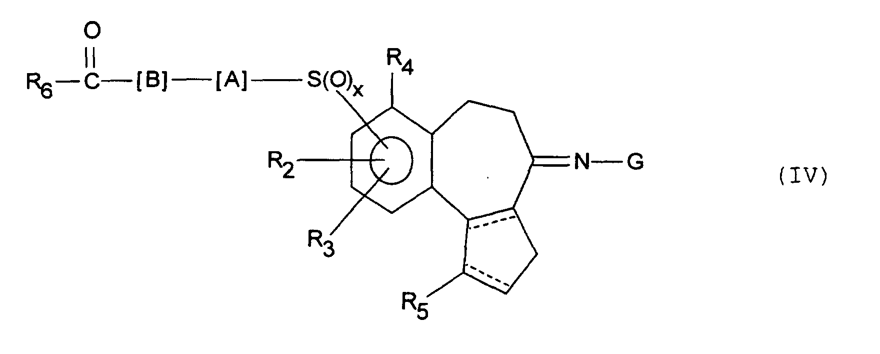
f) compound of formula (IV) which is subjected if appropriate and in an appropriate order:
- to the action of a sulphur oxidizing agent, in order to obtain the compounds of formula (IV) with x = 1 or 2,
- to the action of a base or an acid in order to detach the ester -COR₆ and to obtain the corresponding acid,
- to the action of a dealkylation agent,
- to the action of a deprotection agent of the NH-P function in beta position of CO-R₆ when [B] represents the CH-NHP group,
- to the formation of the NH-SO₂R_{c}, NH-CO₂R_{c}, NHCOR_{c}, NH-SO₂-NH-R_{c}, NH-CO-NHR_{c} group from the corresponding amine in beta position of COR₆,
- to the action of an acid or a base in order to obtain the corresponding salts or to the action of an esterification agent in order to obtain the corresponding esters.

13. Process for the formation of the compounds of formula (I) as defined in claim 1, **characterized in that** a compound of formula (II) as defined in claim 12, is subjected beforehand to the action of a compound of formula (F3) as defined in claim 12, in order to obtain a compound of formula (IIIe): 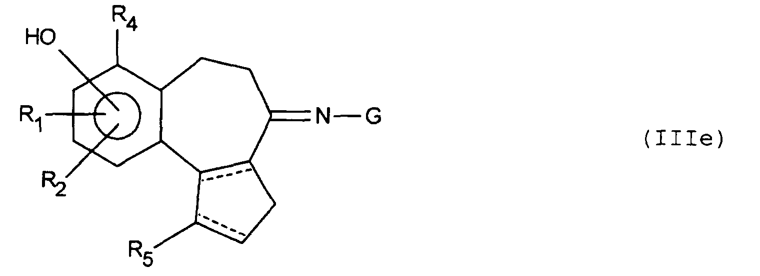 which is then used in the reactions as described in stages a), b), c), d) and if appropriate f) as defined in claim 12.

14. As a medicament, the compounds of formula (I) as defined in any one of claims 1 to 10, as well as their pharmaceutically acceptable addition salts.

15. As a medicament, the compounds of formula (I) as defined in claim 11.

16. The pharmaceutical compositions containing at least one of the medicaments defined in claim 14 or 15 as active ingredient.

17. As novel intermediate products, the compounds of general formulae (IIIa), (IIIb), (IIIc) and (IIId) as defined in claim 12.

## Patentansprüche

1. Verbindungen der allgemeinen Formel (I) 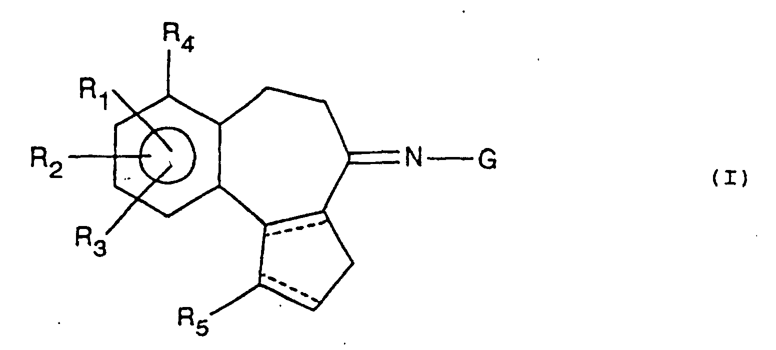 in der R₁ eine Gruppe -S(O)ₓ-[A]-[B]-COR₆ darstellt, in der x gleich 0, 1 oder 2 ist, -[A]- einen linearen oder verzweigten, bivalenten Rest Alkylen, Alkenylen oder Alkinylen mit 1 bis 6 Kohlenstoffatomen bedeutet,
[B] einen Rest Phenyl, einen Rest CH(Z) oder eine einfache Bindung darstellt,
(Z) ein Wasserstoffatom, eine Gruppe (CH₂)₀₋₆-NRaRb, (CH₂)₀₋₆-NH-SO₂-Rc, (CH₂)₀₋₆-NH-CO₂-Rc, (CH₂)₀₋₆-NH-CO-Rc, (CH₂)₀₋₆-NH-SO₂-NH-Rc, (CH₂)₀₋₆-NH-CO-NH-Rc, (CH₂)₀₋₆-CO₂-Rc, (CH₂)₀₋₆-SO₂-Rc, (CH₂)₀₋₆-CO-Rc oder (CH₂)₀₋₆-Rc bedeutet, worin (CH₂)₀₋₆ ein bivalenter Rest Alkylen mit 1 bis 6 Kohlenstoffatomen oder eine einfache Bindung im Fall von (CH₂)₀ ist,
Ra, Rb und Rc ein Wasserstoffatom oder einen Rest (CH₂)₀₋₃-Ar darstellen, worin Ar eine Gruppe Phenyl oder ein Rest (CH₂)₀₋₃-Alk ist, worin Alk einen linearen, verzweigten oder cyclischen Rest Alkyl, Alkenyl oder Alkinyl mit 1 bis 12 Kohlenstoffatomen bedeutet, wobei die Reste Ar und Alk nicht substituiert oder substituiert sein können durch
- Halogen,
- Alkyl, Alkenyl oder Alkinyl mit 1 bis 12 Kohlenstoffatomen,
- Oxo, Cyano, Nitro, Formyl, Carboxy und Carboxyalkyl mit 1 bis 6 Kohlenstoffatomen, Carboxamid,
- Alkoxy mit 1 bis 12 Kohlenstoffatomen,
- Alkylthio mit 1 bis 12 Kohlenstoffatomen,
- Amino, Alkylamino mit 1 bis 12 Kohlenstoffatomen,
- Dialkylamino mit 2 bis 24 Kohlenstoffatomen,
- Aminoalkyl mit 1 bis 12 Kohlenstoffatomen,
- Dialkylaminoalkyl mit 3 bis 25 Kohlenstoffatomen,
- Dialkylaminoalkoxy mit 3 bis 25 Kohlenstoffatomen,
- Hydroxyl, gegebenenfalls acyliert, mit 1 bis 12 Kohlenstoffatomen,
- Acyl mit 1 bis 12 Kohlenstoffatomen oder Benzoyl, gegebenenfalls substituiert durch ein Atom von Chlor, Iod oder Fluor,
- Phenyl, Furyl, Thienyl, Pyridinyl oder Benzyl, gegebenenfalls substituiert durch Halogen, Alkyl, Alkoxy, Alkylthio, Aminoalkyl oder Dialkylamino wie oben angegeben, oder auch
Ra und Rb zusammen mit dem Stickstoffatom, an das sie gebunden sind, einen Heterocyclus darstellen, ausgewählt unter Morpholin, Piperidin, Piperazin, Pyrrolidin, Pyrimidin, Pyridin oder Pyrazin,
- R₆ eine Gruppe -OH, -OCH₃, -OCH₂CH₃, -O-(CH₂)₂-OH, -O-CH₂-CH(OH)-CH₂OH, -O-(CH₂)₂-NH₂, -O-(CH₂)₂-N-(CH₃)₂, -NH₂ oder -O-(CH₂)-phenyl bedeutet,
- R₂ und R₃, gleich oder verschieden, ein Wasserstoffatom, einen Rest Hydroxyl oder einen Rest O-Me darstellen,
- R₄ ein Wasserstoffatom ist,
- R₅ ein Wasserstoffatom ist,
- G darstellt:
entweder einen Rest der Formel G1 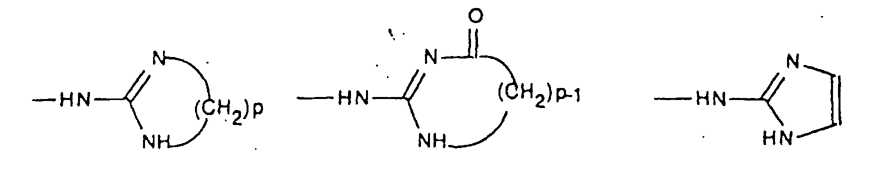 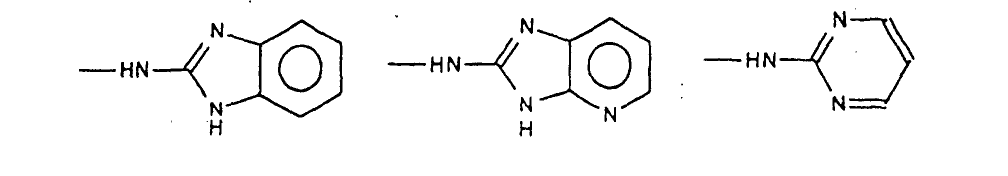 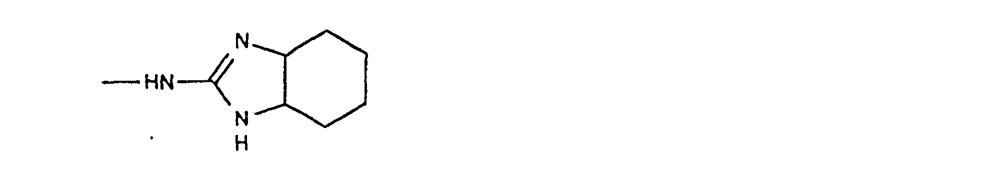
worin p eine ganze Zahl von 2, 3 oder 4 ist,
oder einen Rest NRaRb (Rest G2), worin Ra und Rb wie oben definiert sind,
oder einen Rest -NH-C(=X)-NHRc (Rest G4), worin X ein Schwefelatom oder Sauerstoffatom oder NH bedeutet und Rc wie vorstehend definiert ist,
oder einen Rest -NH-SO₂Rc (Rest G5), worin Rc wie vorstehend definiert ist,
wobei die punktierten Linien eine eventuelle zweite Bindung darstellen, sowie die Additionssalze mit Säuren oder Basen,
und wobei sich R₁, R₂ und R₃ in Position 8, 9 oder 10 der Tricyclus befinden können.

2. Verbindungen der allgemeinen Formel (I) wie in Anspruch 1 definiert, die der allgemeinen Formel (I') 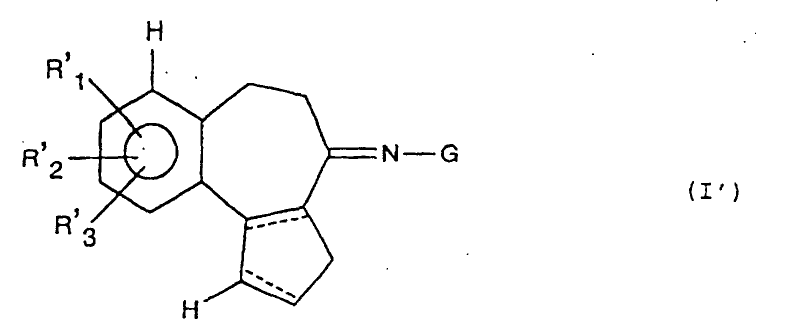 entsprechen,
in der R'₁ eine Gruppe -S(O)ₓ-[A']-[B']-COR'₆ darstellt, in der x gleich 0, 1 oder 2 ist, -[A']- einen bivalenten Rest Alkylen, Alkenylen oder Alkinylen mit 2 bis 6 Kohlenstoffatomen bedeutet, [B'] einen Rest CH(Z') oder eine einfache Bindung darstellt, (Z') ein Wasserstoffatom, eine Gruppe (CH₂)₀₋₆-NRaRb, (CH₂)₀₋₆-NH-SO₂-Rc, (CH₂)₀₋₆-NH-CO₂-Rc, (CH₂)₀₋₆-NH-CO-Rc, (CH₂)₀₋₆-NH-SO₂-NH-Rc, (CH₂)₀₋₆-NH-CO-NH-Rc, (CH₂)₀₋₆-CO₂-Rc, (CH₂)₀₋₆-SO₂-Rc, (CH₂)₀₋₆-CO-Rc oder (CH₂)₀₋₆-Rc bedeutet, Ra, Rb und Rc wie in Anspruch 1 definiert sind, R'₆ eine Gruppe -OH, -OCH₃, -OCH₂CH₃, -O-(CH₂)₂-OH, -O-CH₂-CH(OH) -CH₂OH, -O-(CH₂)₂-NH₂, -O-(CH₂)₂-N-(CH₃)₂, -NH₂ oder -O-(CH₂)-phenyl bedeutet,
R'₂ und R'₃ ein Wasserstoffatom oder einen Rest Methoxy darstellen und G wie in Anspruch 1 definiert ist,
wobei die punktierten Linien eine eventuelle zweite Bindung darstellen, sowie die Additionssalze mit Säuren oder Basen.

3. Verbindungen der allgemeinen Formel (I) wie in Anspruch 1 oder 2 definiert, worin R₆ eine Gruppe -OH oder -OCH₃ darstellt, sowie die Additionssalze mit Säuren oder Basen.

4. Verbindungen der allgemeinen Formel (I) wie in Anspruch 1 definiert, worin R₁ eine Gruppe -S(O)ₓ-(CH₂)₂-CH(Z')-COOH darstellt, in der x gleich 0, 1 oder 2 ist und Z' wie in Anspruch 2 definiert ist, sowie die Additionssalze mit Säuren oder Basen.

5. Verbindungen der allgemeinen Formel (I) wie in Anspruch 4 definiert, worin (Z') die Gruppe (CH₂)₀₋₆-NH-CO₂-Rc oder (CH₂)₀₋₆-NH-Rb bedeutet und Rb und Rc wie in Anspruch 1 definiert sind, sowie die Additionssalze mit Säuren oder Basen.

6. Verbindungen der allgemeinen Formel (I) wie in Anspruch 5 definiert, worin (Z') NHCO₂Rc oder NHRb ist, sowie die Additionssalze mit Säuren oder Basen.

7. Verbindungen der allgemeinen Formel (I) wie in Anspruch 6 definiert, worin Rb und Rc Gruppen (CH₂)₀₋₃-Ar oder (CH₂)₀₋₃-Alk sind und Ar und Alk wie in Anspruch 1 definiert sind, sowie die Additionssalze mit Säuren oder Basen.

8. Verbindungen der allgemeinen Formel (I) wie in irgendeinem der Ansprüche 1 bis 7 definiert, worin G eine Gruppe G4 der allgemeinen Formel -NH-C-(=NH)-NHRc darstellt und Rc wie in Anspruch 1 definiert ist, sowie die Additionssalze mit Säuren oder Basen.

9. Verbindungen der allgemeinen Formel (I) wie in Anspruch 8 definiert, worin Rc ein Wasserstoffatom ist, sowie die Additionssalze mit Säuren oder Basen.

10. Verbindungen der allgemeinen Formel (I) wie in Anspruch 1 definiert, worin G die Gruppe 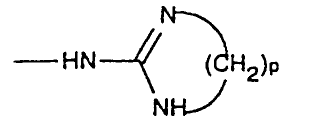 bedeutet und p eine ganze Zahl von 2, 3 oder 4 ist, sowie die Additionssalze mit Säuren oder Basen.

11. Verbindungen der Formel (I) wie in Anspruch 1 definiert, mit den folgenden Namen:
- S-{4-[(4,5-Dihydro-1H-imidazol-2-yl)-hydrazono]-9,10-dimethoxy-1,2,3,4,5,6-hexahydro-8-benz[e]-azulenyl}-N-[(phenylmethoxy)-carbonyl]-DL-homocystein
- 4-{[4-[(4,5-Dihydro-1H-imidazol-2-yl)-hydrazono]-9,10-dimethoxy-1,2,3,4,5,6-hexahydro-8-benz[e]-azulenyl]-sulfinyl}-2-{[(phenylmethoxy)-carbonyl]-amino}-butansäure
- 4-{[4-[(4,5-Dihydro-1H-imidazol-2-yl)-hydrazono]-9,10-dimethoxy-1,2,3,4,5,6-hexahydro-8-benz[e]-azulenyl]-sulfonyl}-2-{[(phenylmethoxy)-carbonyl]-amino}-butansäure.

12. Verfahren zur Herstellung der Verbindungen der Formel (I) wie in Anspruch 1 definiert, umfassend die folgenden Stufen:
a) Einwirkung einer Verbindung der Formel (F1) 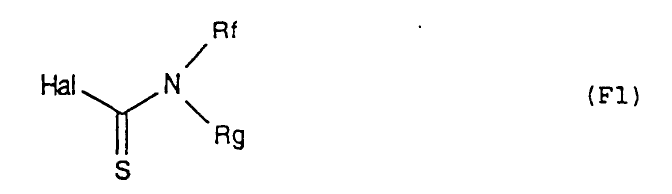 in der Hal ein Atom von Chlor oder Brom darstellt und Rf und Rg einen Rest Alkyl mit 1 bis 4 Kohlenstoffatomen bedeuten, in Anwesenheit einer Base auf eine Verbindung der Formel (II) 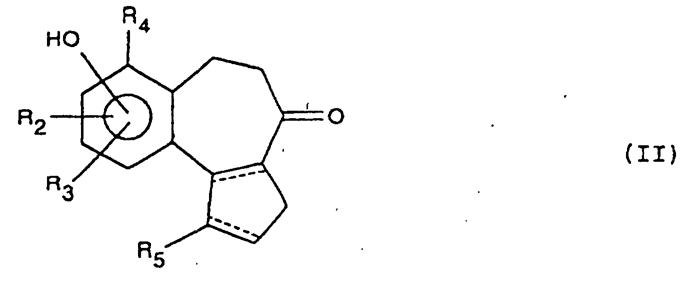 in der R₂, R₃, R₄ und R₅ so sind wie in Anspruch 1 beschrieben, mit Ausnahme des Wertes Hydroxyl, um die Verbindung der Formel (IIIa) 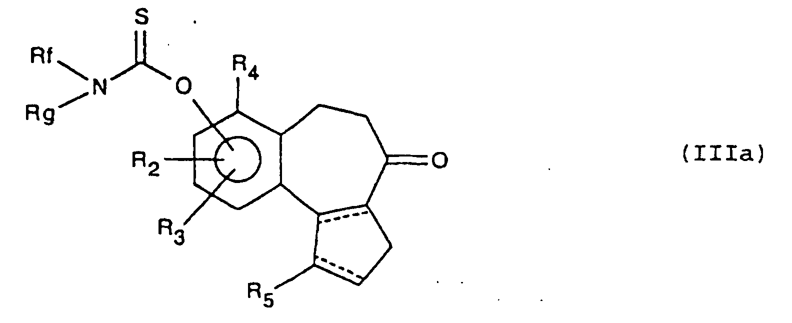 zu erhalten,
b) Pyrolyse der Verbindung der Formel (IIIa) um die Verbindung der Formel (IIIb) 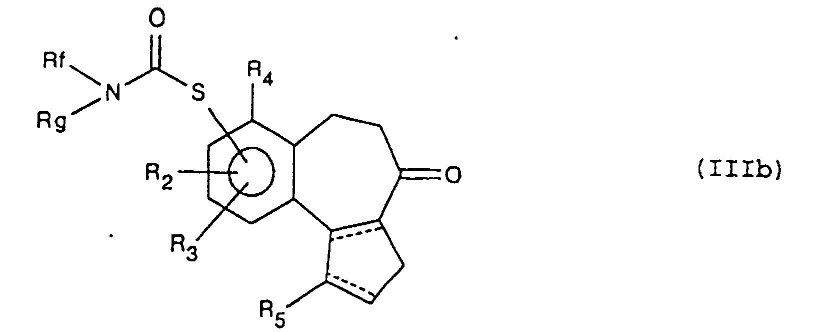 zu erhalten,
c) Hydrolyse der Verbindung der Formel (IIIb) im basischen Medium und anschließende Neutralisation, um das Thiol der Formel (IIIc) 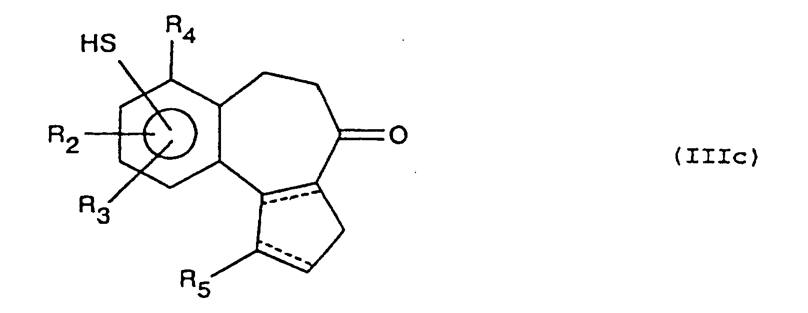 zu erhalten,
d) Einwirkung einer Verbindung der Formel (F2)
Hal-[A]-[B]-COR₂ (F2)
in der Hal, [A], [B] und R₆ wie in Anspruch 1 definiert sind und [B] ebenfalls die Gruppe -CH-NHP darstellen kann, worin P eine Schutzgruppe für die Aminfunktion ist, auf die Verbindung der Formel (IIIc), um eine Verbindung der Formel (IIId) 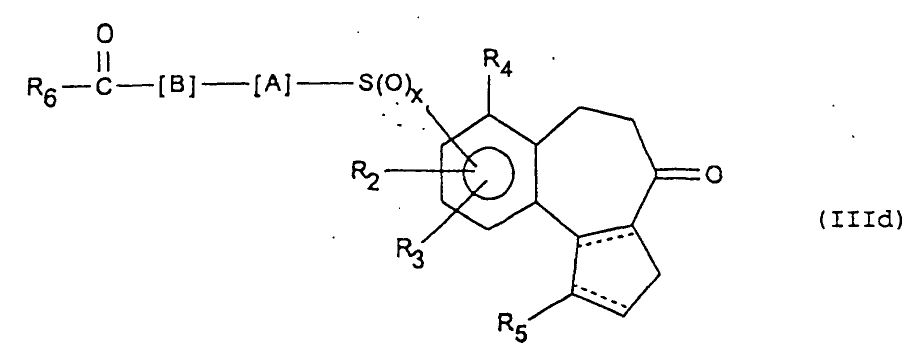 zu erhalten, in der x gleich 0 ist,
e) Einwirkung einer Verbindung der Formel (F3)
H₂N-G (F3)
in der G wie in Anspruch 1 definiert ist, auf die Verbindung der Formel (IIId), um eine Verbindung der Formel (IV) 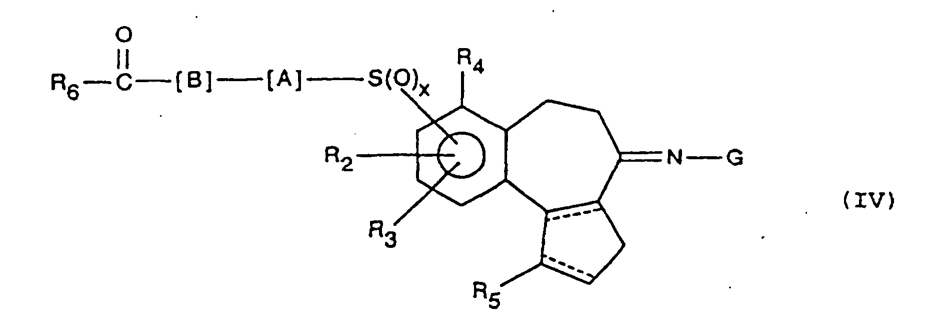 zu erhalten, die den Verbindungen der Formel (I) entspricht mit x = 0,
f) und man die Verbindung der Formel (IV) gegebenenfalls in irgendeiner Reihenfolge unterzieht:
- der Einwirkung eines Oxidationsmittels für den Schwefel, um die Verbindungen der Formel (IV) mit x = 1 oder 2 zu erhalten,
- der Einwirkung einer Base oder einer Säure, um den Ester -COR₆ zu spalten und die entsprechende Säure zu erhalten,
- der Einwirkung eines Mittels zur Dealkylierung,
- der Einwirkung eines Mittels zur Abspaltung der Schutzgruppe von der Funktion NH-P in beta von CO-R₆, wenn [B] die Gruppe CH-NHP darstellt,
- der Bildung der Gruppe NH-SO₂R_{c}, NH-CO₂R_{c}, NHCOR_{c}, NH-SO₂-NH-R_{c}, NH-CONH-R_{c}, ausgehend von dem entsprechenden Amin in beta von COR₆,
- der Einwirkung einer Säure oder einer Base, um die entsprechenden Salze zu erhalten, oder der Einwirkung eines Mittels zur Veresterung, um die entsprechenden Ester zu erhalten.

13. Verfahren zur Herstellung der Verbindungen der Formel (I) wie in Anspruch 1 definiert, **dadurch gekennzeichnet, daß** man zuerst eine Verbindung der Formel (II), wie in Anspruch 12 definiert, der Einwirkung einer Verbindung der Formel (F3), wie in Anspruch 12 definiert, unterzieht, um eine Verbindung der Formel (IIIe) 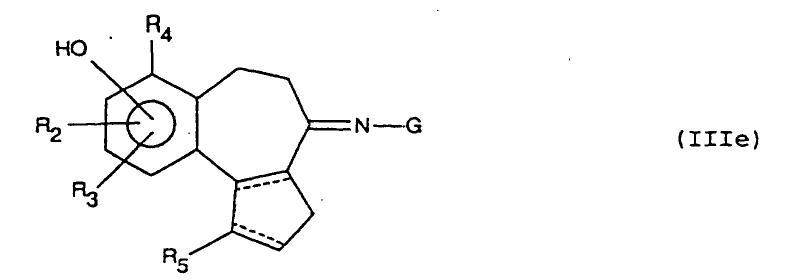 zu erhalten, die man anschließend den Reaktionen unterwirft, wie sie in den Stufen a), b), c), d) und gegebenenfalls f) beschrieben und in Anspruch 12 definiert sind.

14. Als Arzneimittel die Verbindungen der Formel (I) wie in irgendeinem der Ansprüche 1 bis 10 definiert, sowie ihre pharmazeutisch akzeptablen Additionssalze.

15. Als Arzneimittel die Verbindungen der Formel (I) wie in Anspruch 11 definiert.

16. Pharmazeutische Zusammensetzungen, umfassend als Wirkstoff mindestens eines der in Anspruch 14 oder 15 definierten Arzneimittel.

17. Als Zwischenprodukte die Verbindungen der allgemeinen Formeln (IIIa), (IIIb), (IIIc) und (IIId) wie in Anspruch 12 definiert.
